(19) Europäisches Patentamt
European Patent Office
Office européen des brevets

(11) Publication number: **0 209 848 A2**

(12) **EUROPEAN PATENT APPLICATION**

(21) Application number: 86109765.7

(22) Date of filing: 16.07.86

(51) Int. Cl.[4]: C 07 K 5/06
C 07 F 9/30, C 07 F 9/38
C 07 F 9/32, C 07 F 9/40
C 07 F 9/65, A 61 K 31/66
A 61 K 37/02

(30) Priority: 24.07.85 US 758010

(43) Date of publication of application:
28.01.87 Bulletin 87/5

(84) Designated Contracting States:
AT BE CH DE FR GB IT LI LU NL SE

(71) Applicant: MERCK & CO. INC.
126, East Lincoln Avenue P.O. Box 2000
Rahway New Jersey 07065(US)

(72) Inventor: Patchett, Arthur A.
1090 Minisink Way
Westfield New Jersey 07090(US)

(72) Inventor: Parsons, William H.
2172 Oliver Street
Rahway New Jersey 07065(US)

(72) Inventor: Greenlee, William J.
115 Herrick Avenue
Teaneck New Jersey 07666(US)

(74) Representative: Abitz, Walter, Dr.-Ing. et al,
Abitz, Morf, Gritschneder, Freiherr von Wittgenstein
Postfach 86 01 09
D-8000 München 86(DE)

(54) **Enzyme inhibitors.**

(57) Peptides of the formula

A–B–D–E–G

are disclosed wherein

A is, e.g., benzyloxycarbonyl, t-butoxycarbonyl, 1-naphthyloxyacetyl or 1-naphthylacetyl;

B is, e.g., phenylalanine, 3-(1-naphthyl)alanine, tryptophan or homophenylalanine;

D is, e.g., histidine, lysine or phenylalanine;

E is, e.g., one of the following formulae:

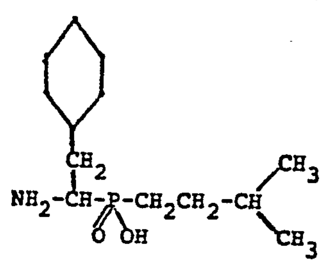

or

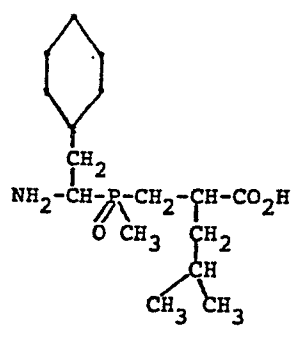

and G is, e.g., –OH, –OEt, $-NH_2$.

Those compounds inhibit enzymes and can be used as pharmaceutical agents.

EP 0 209 848 A2

TITLE OF THE INVENTION

ENZYME INHIBITORS

BACKGROUND OF THE INVENTION

1. Field Of The Invention

The present invention is concerned with novel peptides which inhibit enzymes (renin and angiotensin converting enzyme).

The present invention is also concerned with pharmaceutical compositions containing the novel peptides of the present invention as active ingredients, with methods of treating hypertension and congestive heart failure, with diagnostic methods which utilize the novel peptides of the present invention, and with methods of preparing the novel peptides of the present invention.

2. Brief Description Of The Prior Art

Small peptide inhibitors of renin have been reported by Kokubu *et al*. (*Biochem. Biophys. Res. Communs*, *118*, 929 (1984)) and by Fehrentz *et al*.

(Febs. Letts., 167, 273 (1984)), who described peptide aldehyde inhibitors as short as tripeptide analogs. R. Matsuda et al. in European Patent Application 128,762 (Sankyo Co., Ltd., 1984) also disclose tripeptide aldehydes as renin inhibitors.

In the present invention are disclosed small peptides of four amino acids or the equivalent, which incorporate phosphorus-containing elements as the carboxyl-terminal or next-to carboxyl-terminal component. These peptides are potent inhibitors of renin.

Some of the peptides disclosed in the present invention also inhibit angiotensin-converting enzyme ACE. Peptides containing aminophosphonic acids as the carboxyl-terminal components, which are inhibitors of ACE have previously been reported by Y. Kido et al., in J. Antibiot. (Tokyo), 37, 965-9 (1984) and in Japanese Patent J59187-790-A (Kyowa Hakko Kogyo KK) (1984). Also, phosphinyl-alkanoyl substituted proline analogs which inhibit ACE have previously been described by E. Petrillo et al., U. S. Patents No 4,168,267; 4,337,201 (E. R. Squibb and Sons, 1979, 1982).

## STATEMENT OF THE INVENTION

Peptides of the formula A-B-D-E-G containing a phosphorus bearing moiety and their use as enzyme inhibitors.

DETAILED DESCRIPTION OF THE INVENTION

The present invention is embodied in peptides of the following formula:

A-B-D-E-G

I

wherein:

A is hydrogen, or R-, RCO- or $RSO_2$-, where R is alkyl, cycloalkyl, aryl, heterocyclic, heterocyclic alkyl, heterocyclic aryl, aryloxy alkyl, heterocyclic aryloxy alkyl, aryl alkyl, heterocyclic aryl alkyl, heterocyclic oxyalkyl, and R- may be substituted by amino, hydroxy, alkyl, halo, and alkoxy groups.

B and D can independently be absent or can be:

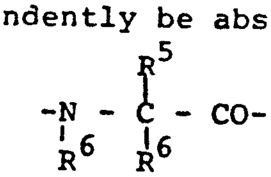

II

E is

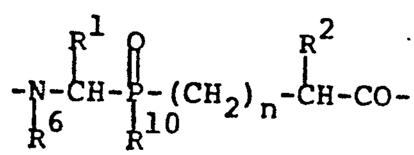

III

or

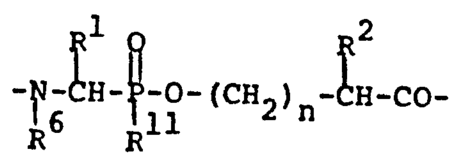

IV

or, in the case where G is absent, E may be:

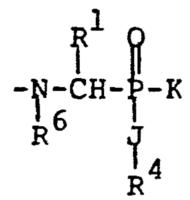

V

E may also be:

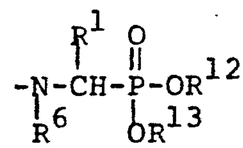

VI

G is $R^3$ or is:

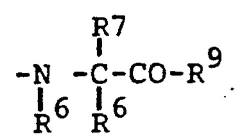

J is -O- or, in the case where $R^4$ does not equal hydrogen, may be absent.

K is hydrogen, alkyl, alkenyl, alkynyl, cycloalkyl, cycloalkenyl, aryl alkyl, cycloalkyl alkyl, heterocyclic, heterocyclic alkyl, each of which may be substituted by up to three members selected from amino, alkyl amino, dialkyl amino, trialkyl ammonium, hydroxy, alkoxy, aryloxy, arylalkoxy, alkanoylamino, aroylamino, or halo.

K may also be

$$-(CH_2)_{n'}-CO-R^3$$

where n' is 3 to 5 and $R^3$ is as defined below.

K may also be of the formula:

$$-(CH_2)_p-NH-L$$

where p is 1 to 2 and

L is $-\underset{R^5}{CH}-CO-R^3$,

$-CO-\underset{R^5}{CH}-NH_2$, or A as defined above.

$R^1$ is alkyl, alkenyl, alkynyl, cycloalkyl, cycloalkenyl, cycloalkyl alkyl, aryl alkyl, heterocyclic, heterocyclic alkyl, each of which may be substituted by up to three groups selected from alkyl, halo, amino and alkoxy groups.

n is 0, 1.

$R^2$ is hydrogen, alkyl, alkenyl, alkynyl, cycloalkyl, cycloalkenyl, cycloalkyl alkyl, aryl, aryl alkyl, heterocyclic, heterocyclic alkyl, each of which may be substituted by up to three groups selected from alkyl, hydroxy, halo, amino, alkylamino, dialkylamino, and alkoxy groups.

$R^3$ is OH, $NH_2$, $NHR^3_a$, $NR^3_aR^3_b$, $OR^3_c$ where $R^3_a$, $R^3_b$, and $R^3_c$ are separately alkyl, alkenyl, alkynyl, cycloalkyl, cycloalkenyl, cycloalkyl alkyl, aryl, aryl alkyl, heterocyclic, heterocyclic

alkyl, each of which may be substituted by up to three groups selected from amino, alkyl amino, dialkyl amino, trialkyl ammonium, hydroxy, alkoxy, aryloxy, aryl alkoxyl, or halo.

$R_c^3$ may also be $R_d^3$-CO-V-$CR_e^3R_f^3$ wherein $R_d^3$ is alkyl or aryl; $R_e^3$ and $R_f^3$ are hydrogen or alkyl; V is -O- or -NH-.

$R^4$ is hydrogen, alkyl, alkenyl, alkynyl, aryl, arylalkyl, each of which may be substituted by amino, alkyl amino, dialkyl amino, trialkyl ammonium, hydroxy, alkoxy, halo or alkyl groups. $R^4$ may also be $R_a^4$-CO-V-$CR_b^4R_c^4$ wherein $R_a^4$ is alkyl or aryl; $R_b^4$ and $R_c^4$ are hydrogen or alkyl; V is -O- or -NH-.

$R^5$ is hydrogen, alkyl, alkenyl, alkynyl, cycloalkyl, cycloalkenyl, aryl, cycloalkyl alkyl, aryl alkyl, heterocyclic, heterocyclic alkyl, aryloxy alkyl, heterocyclic oxy alkyl, heterocyclic oxy oxy, each of which may be substituted by amino, alkyl amino, dialkyl amino, trialkyl ammonium, hydroxy, alkoxy, carboxy, alkoxycarbonyl, alkylthio, arylthio, thiol, guanidino, carboxamido and alkanoylamino groups.

$R^6$ is hydrogen, methyl.

$R^7$ can be $R^5$ and taken together with $NR^8$ may be a cyclic amino acid of formulas:

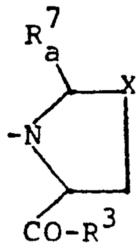 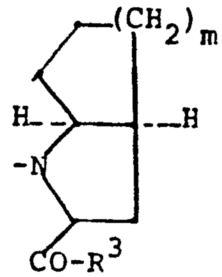 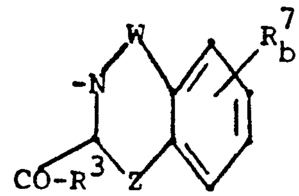

where $R^7_a$ is hydrogen, phenyl, hydroxyphenyl; X is -S- or $-CH_2-$ or $-CH-R^7_b$; m is 1 or 2; and $R^7_b$ is cyclohexyl, phenylthio; W and Z are bonds or $-CH_2-$, and $R^3$ is as defined above.

$R^8$ is hydrogen, methyl and cycloalkyl including cyclopentyl and indanyl. When $R^8$ is cycloalkyl, $R^6$ and $R^7$ are hydrogen.

$R^9$ is hydroxy, $OR^3_a$, $-NH_2$, $-NHR^3_a$, $NR^3_aR^3_b$, where $R^3_a$ and $R^3_b$ are as defined above. When A and B are both absent, $R^9$ can be 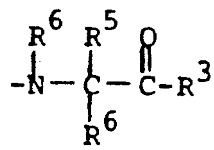 where $R^3$, $R^5$ and $R^6$ are as defined above.

$R^{10}$ is alkyl, aryl alkyl, heterocyclic, heterocyclic alkyl, where each of these may be substituted by up to three groups selected from amino, alkyl amino, dialkyl amino, trialkyl ammonium, hydroxy, alkoxy, thiol, carboxamido, and alkanoylamino or aroylamino groups.

$R^{11}$ is hydrogen or $-OR^4$, where $R^4$ is as defined above.

$R^{12}$ and $R^{13}$ may independently be hydrogen, alkyl, alkenyl, alkynyl, aryl, aryl alkyl, each of which may be substituted by up to three groups selected from hydroxy, alkyl, amino, alkyl amino, dialkyl amino, trialkyl ammonium, halo, alkanoylamino, aroylamino, alkoxy, and aryloxy groups.

In the above definitions, the terms alkyl, alk, alkenyl, and alkynyl are intended to include hydrocarbon groups of up to 8 carbon atoms which groups may be straight chain or branched chain. Preferred alkyl groups have 1-4 carbon atoms. Preferred alkenyl and alkynyl groups have 3-6 carbon atoms.

The term halo means fluoro, chloro, bromo and iodo.

Aryl represents hydrocarbons of up to 10 carbon atoms. Examples of the aryl moiety are phenyl, naphthyl, biphenyl and cycloalkyl-fused derivatives thereof such as indanyl and tetralinyl and the like.

The heterocyclic substituent recited above represents any moiety which contains 5- or 6-membered ring containing from one to three heteroatoms selected from the group consisting of nitrogen, oxygen and sulfur wherein the nitrogen and sulfur heteroatoms may optionally be quaternized, including any bicyclic group in which any of the above heterocyclic rings is fused to a benzene ring, said heterocyclic group being saturated or unsaturated. Heterocyclic substituents in which nitrogen is the heteroatom are preferred; and of these, those

containing a single nitrogen atom are preferred. Fully saturated heterocyclic substituents are also preferred. Thus, piperidine is a preferred heterocyclic substituent. Other preferred heterocyclic substituents are pyrrolidinyl, imidazolidinyl, morpholinyl, tetrahydrofuranyl, thienyl, pyrimidinyl, imidazolyl, indolyl, quinolinyl, isoquinolinyl, benzothienyl and the like.

Cycloalkyl or cycloalkenyl groups contain up to 12 carbon atoms and may be bridged. They are exemplified by cyclopropyl, cyclopentyl, cyclohexyl, cyclohexenyl, nobornenyl, adamantyl, bicyclo[3.3.0]-octanyl, perhydronaphthyl and the like.

In the above definitions, when A is RCO, and A is alkyl, aryl, aryl alkyl, heterocyclic or heterocyclic alky, these groups being optionally substituted by amino, alkanoylamino, hydroxy, alkyl, alkoxy, alkoxycarbonyl, halo, or nitro; and B is absent and D is

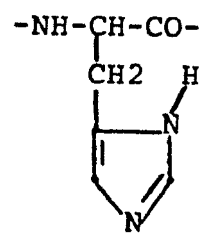

and E is of the formula V where $R^6$ is hydrogen; $R^1$ is isobutyl or sec-butyl and K is alkyl or alkoxy substituted with at least one hydroxy, mercapto, amino, carbamoyl, formyl, aryl or heterocyclic substituent, then if J is -O-, $R^4$ may not equal hydrogen. When these same definitions above for A, B, and D are in effect, then for E of the formula VI, $R^{12}$ and $R^{13}$ may not both be hydrogen.

The peptides of the present invention may also be described in terms of common amino acid components and closely related analogs thereof, in accordance with formula (I):

A-B-D-E-G

(I)

wherein A has the same meaning as described above: A and B can each be, for example, Ala, Leu, Phe, Tyr, Trp, Met, HomoPhe, BishomoPhe, HomoTyr, HomoTrp, 3-(1-Naphthyl)Ala, 3-(2-Naphthyl)Ala, 5-Methoxy Trp, N-Methyl Phe, N-Methyl-HomoPhe, α-methylPhe.

It will be understood that closely related analogs of the above common amino acids, for examples, aliphatic amino acids in addition to Ala, Val, Leu, and Ile, such as alpha amino butyric acid (Abu) are included in the broad description of the peptides of the present invention represented by Formula (I) and its definitions.

Preferred A units include benzyloxycarbonyl, t-butoxycarbonyl, 1-naphthyloxyacetyl and 1-naphthylacetyl.

Preferred B amino acid units include phenylalanine, 3-(1-naphthyl)alanine, tryptophan and homophenylalanine.

Preferred D amino acid units include histidine, lysine and phenylalanine.

Preferred E units include those with the following formulae:

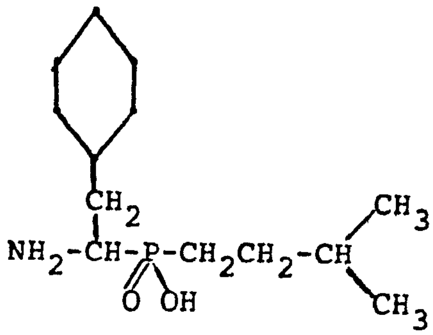

VIII

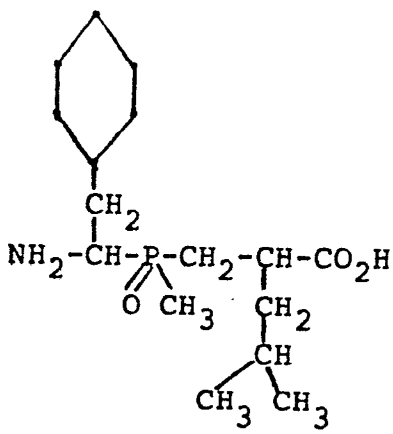

IX

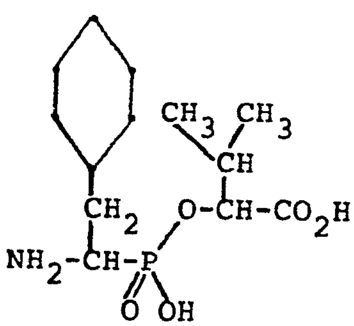

X

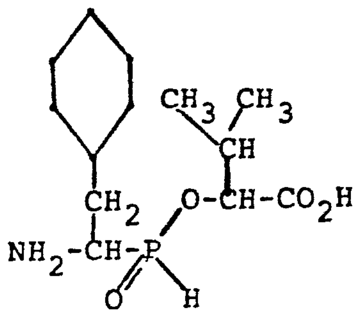

XI

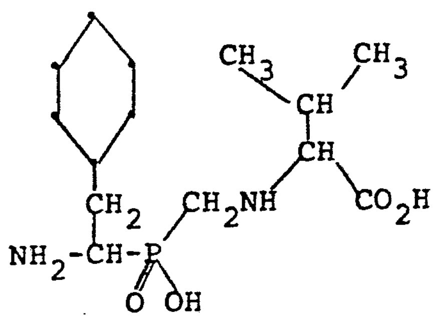

VII

Preferred G units include -OH, -OEt, $-NH_2$.

The amino acid units have asymmetric centers and occur as racemates, racemic mixtures and as individual diastereomers. All isomeric forms are included in the present peptides. In general, the preferred chiral forms of amino acid units B and D are the (L) forms. The stereocenters present in the E unit of the peptides of formula I are in general of the chirality which corresponds to the naturally-occurring (L) amino acids. Thus, for example, the unit E of the formula below possesses the stereochemistry (R and S) shown in the preferred form:

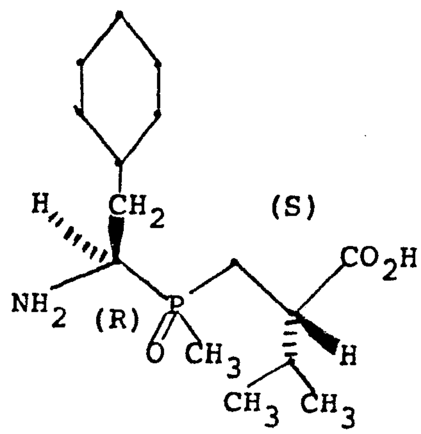

In cases where the phosphorous atom of the B unit represents a chiral center, as in structures IX, X and XI above, both chiral forms are preferred.

The following are illustrative examples of peptides of the present invention:

1. [N-(N-(N-Carbobenzoxy-2-amino-3-(1-naphthyl)-propionyl)histidyl)-1-amino-2-cyclohexylethyl] 2-carboxy-4-methylpentyl methyl phosphine oxide
2. [N-(N-(N-Carbobenzoxy-2-amino-3-(1-naphthyl)-propionyl)histidyl)-1-amino-2-cyclohexylethyl] 2-carboxy-3-methylbutylphosphine oxide
3. 2-Carboxy-3-methylbutyl [N-(N-(N-t-butoxycarbonyl-2-amino-3-(1-naphthyl)propionyl)histidyl)-1-amino-2-cyclohexylethyl]phosphinate
4. 2-Carbomethoxy-4-methylpentyl [N-(N-(N-carbobenzoxy-2-amino-3-(1-naphthyl)propionyl)histidyl)-1-amino-2-cyclohexylethyl] methylphosphinate
5. [N-(N-(N-Carbobenzoxy-2-amino-3-(1-naphthyl)-propionyl)histidyl)-1-amino-2-cyclohexylethyl]-pho: ›honic acid
6. Ethyl [N-(N-(N-t-butoxycarbonyl-2-amino-3-(1-naphthyl)propionyl)histidyl)-1-amino-2-cyclohexylethyl]phosphonate

7. 2-Carboxy-4-methylpentyl methyl [N-(N-carbobenzoxy-phenylalanyl-histidyl)-1-amino-2-cyclohexylethyl] phosphonate
8. [N-(N-Carbobenzoxy-phenylalanyl-histidyl)-1-amino-2-cyclohexylethyl] 2-(N-benzyl)carboxamido-4-methylpentyl benzyl phosphine oxide
9. 2-Carboxamido-4-methylpentyl [N-(N-carbobenzoxy-phenylalanyl-phenylalanyl)-1-amino-2-cyclohexylethyl]phosphinate
10. [N-(N-(N-Carbobenzoxy-2-amino-3-(1-naphthyl)-propionyl)histidyl)-1-amino-2-cyclohexylethyl] 3-methylbutylphosphinic acid
11. Methyl [N-(N-(N-carbobenzoxy-2-amino-3-(1-naphthyl)propionyl)histidyl)-1-amino-2-cyclohexylethyl] 4-methylpentylphosphinate
12. [N-(N-(N-Carbobenzoxy-2-amino-3-(1-naphthyl)-propionyl)histidyl)-1-amino-2-cyclohexylethyl] aminomethylphosphinic acid
13. [N-(N-(N-Carbobenzoxy-2-amino-3-(1-naphthyl)-propionyl)histidyl)-1-amino-2-cyclohexylethyl] (N-benzoyl)aminomethylphosphinic acid
14. [N-(N-Carbobenzoxy-histidyl)-1-amino-2-cyclohexylethyl] 4-methylpentylphosphinic acid
15. [N-(Phenylalanyl)-1-amino-2-cyclohexylethyl]-3-methylbutylphosphinic acid
16. [N-(Lysyl)-1-amino-2-cyclohexylethyl]-3-methylbutylphosphinic acid
17. [N-(N-1-Naphthyloxyacetyl-lysyl)-1-amino-2-cyclohexylethyl] (n-benzyl)aminomethylphosphinic acid
18. Methyl [N-(N-(N-carbobenzoxy-2-amino-3-(1-naphthyl)propionyl)histidyl)-1-amino-2-cyclohexylethyl] 4-phenylpentylphosphinate

The present invention includes the pharmaceutically acceptable salts of the Formula I compounds. These salts include acid addition salts such as: acetate, adipate, alginate, aspartate, benzoate, benzenesulfonate, bisulfate, butyrate, citrate, camphorate, camphorsulfonate, cyclopentane-propionate, digluconate, dodecylsulfate, ethane-sulfonate, fumarate, glucoheptanoate, glycero-phosphate, hemisulfate, heptanoate, hexanoate, hydrochloride, hydrobromide, hydroiodide, 2-hydroxyethanesulfonate, lactate, maleate, methanesulfonate, 2-naphthalenesulfonate, nicotinate, oxalate, pamoate, pectinate, persulfate, 3-phenylpropionate, picrate, pivalate, propionate, succinate, tartrate, thiocyanate, tosylate, and undecanoate. The base salts also include ammonium salts, alkali metal salts such as sodium and potassium salts, alkaline earth metal salts such as calcium and magnesium salts, salts with organic bases such as dicyclohexylamine salts, N-methyl-D-glucamine, and salts with amino acids such as arginine, lysine, and so forth. Also, the basic nitrogen-containing groups can be quaternized with such agents as lower alkyl halides, such as methyl, ethyl, propyl, and butyl chloride, bromides and iodides; dialkyl sulfates like dimethyl, diethyl, dibutyl; and diamyl sulfates, long chain halides such as decyl, lauryl, myristyl and stearyl chlorides, bromides and iodides, aralkyl halides like benzyl and phenethyl bromides and others. Water or oil-soluble or dispersible products are thereby obtained. Conventional methods of preparing these salts may be used.

The present invention is also directed to combinations of the novel enzyme-inhibitory peptides of Formula I with one or more antihypertensive agents selected from the group consisting of diuretics, α and/or ß-adrenergic blocking agents, CNS-acting antihypertensive agents, adrenergic neuron blocking agents, vasodilators, angiotensin I converting enzyme inhibitors, calcium channel blockers and other antihypertensive agents.

For example, the compounds of this invention can be given in combination with such compounds or salt or other derivative forms thereof as:

Diuretics: acetazolamide; amiloride; bendroflumethiazide; benzthiazide; bumetanide; chlorothiazide; chlorthalidone; cyclothiazide; ethacrynic acid; furosemide; hydrochlorothiazide; hydroflumethiazide; indacrinone (racemic mixture, or as either the (+) or (-) enantiomer alone, or a manipulated ratio, e.g., 9:1 of said enantiomers, respectively); metolazone; methyclothiazide; muzolimine; polythiazide; quinethazone; sodium ethacrynate; sodium nitroprusside; spironolactone; ticrynafen; triamterene; trichlormethiazide;

α-Adrenergic Blocking Agents: dibenamine; phentolamine; phenoxybenzamine; prazosin; tolazoline;

ß-Adrenergic Blocking Agents: atenolol; metoprolol; nadolol; propranolol; timolol;

((±)-2-[3-(tert-butylamino)-2-hydroxypropoxy]-2-furananilide) (ancarolol);

(2-acetyl-7-(2-hydroxy-3-isopropylaminopropoxy)benzofuran HCl) (befunolol);

((±)-1-(isopropylamino)-3-(p-(2-cyclopropylmethoxyethyl)-phenoxy)-2-propranol HCl) (betaxolol);

(1-[(3,4-dimethoxyphenethyl)amino]-3-(m-tolyloxy)-2-propanol HCl) (bevantolol);

(((±)-1-(4-((2-isopropoxyethoxy)methyl)phenoxy)-3-isopropylamino-2-propanol)fumarate) (bisoprolol);

(4-(2-hydroxy-3-[4-(phenoxymethyl)-piperidino]-propoxy)-indole);

(carbazolyl-4-oxy-5,2-(2-methoxyphenoxy)-ethylamino-2-propanol);

(1-((1,1-dimethylethyl)amino)-3-((2-methyl-1H-indol-4-yl)oxy)-2-propanol benzoate) (bopindolol);

(1-(2-exobicyclo[2.2.1]-hept-2-ylphenoxy)-3-[(1-methylethyl)-amino]-2-propanol HCl) (bornaprolol);

(o-[2-hydroxy-3-[(2-indol-3-yl-1,1-dimethylethyl)-amino]propoxy]benzonitrile HCl) (bucindolol);

(α-[(tert.butylamino)methyl]-7-ethyl-2-benzofuranmethanol) (bufuralol);

(3-[3-acetyl-4-[3-(tert.butylamino)-2-hydroxypropyl]-phenyl]-1,1-diethylurea HCl) (celiprolol);

((±)-2-[2-[3-[(1,1-dimethylethyl)amino]-2-hydroxypropoxy]phenoxy]-N-methylacetamide HCl) (cetamolol);

(2-benzimidazolyl-phenyl(2-isopropylaminopropanol));

((±)-3'-acetyl-4'-(2-hydroxy-3-isopropylaminopropoxy)-acetanilide HCl) (diacetolol);

(methyl-4-[2-hydroxy-3-[(1-methylethyl)aminopropoxy]]-benzenepropanoate HCl) (esmolol);

(erythro-DL-1-(7-methylindan-4-yloxy)-3-isopropylaminobutan-2-ol);

(1-(tert.butylamino)-3-[O-(2-propynyloxy)phenoxy]-2-propanol (pargolol);

(1-(tert.butylamino)-3-[o-(6-hydrazino-3-pyridazinyl)-phenoxy]-2-propanol diHCl) (prizidilol);

((-)-2-hydroxy-5-[(R)-1-hydroxy-2-[(R)-(1-methyl-3-phenylpropyl)amino]ethyl]benzamide);

(4-hydroxy-9-[2-hydroxy-3-(isopropylamino)-propoxy]-7-methyl-5H-furo[3,2-g][1]-benzopyran-5-one) (iprocrolol);

((-)-5-(tert.butylamino)-2-hydroxypropoxy]-3,4-dihydro-1-(2H)-naphthalenone HCl) (levobunolol);

(4-(2-hydroxy-3-isopropylamino-propoxy)-1,2-benziso-thiazole HCl);

(4-[3-(tert.butylamino)-2-hydroxypropoxy]-N-methyliso-carbostyril HCl);

((±)-N-2-[4-(2-hydroxy-3-isopropyl aminopropoxy)-phenyl]ethyl-N'-isopropylurea) (pafenolol);

(3-[[(2-trifluoroacetamido)ethyl]amino]-1-phenoxy-propan-2-ol);

(N-(3-(o-chlorophenoxy)-2-hydroxypropyl)-N'-(4'-chloro-2,3-dihydro-3-oxo-5-pyridazinyl)ethylenediamine);

((±)-N-[3-acetyl-4-[2-hydroxy-3-[(1-methylethyl)amino]-propoxy]phenyl]butanamide) (acebutolol);

((±)-4'-[3-(tert-butylamino)-2-hydroxypropoxy]spiro-[cyclohexane-1,2'-indan]-1'-one) (spirendolol);

(7-[3-[[2-hydroxy-3-[(2-methylindol-4-yl)oxy]propyl]-amino]butyl]thiophylline) (teoprolol);

((±)-1-tert.butylamino-3-(thiochroman-8-yloxy)-2-propanol) (tertatolol);

((±)-1-tert.butylamino-3-(2,3-xylyloxy)-2-propanol HCl) (xibenolol);

(8-[3-(tert.butylamino)-2-hydroxypropoxy]-5-methylcoumarin) (bucumolol);

(2-(3-(tert.butylamino)-2-hydroxy-propoxy)benzonitrile HCl) (bunitrolol);

((±)-2'-[3-(tert-butylamino)-2-hydroxypropoxy-5'-fluorobutyrophenone) (butofilolol);

(1-(carbazol-4-yloxy)-3-(isopropylamino)-2-propanol) (carazolol);

(5-(3-tert.butylamino-2-hydroxy)propoxy-3,4-dihydrocarbostyril HCl) (carteolol);

(1-(tert.butylamino)-3-(2,5-dichlorophenoxy)-2-propanol) (cloranolol);

(1-(inden-4(or 7)-yloxy)-3-(isopropylamino)-2-propanol HCl) (indenolol);

(1-isopropylamino-3-[(2-methylindol-4-yl)oxy]-2-propanol) (mepindolol);

(1-(4-acetoxy-2,3,5-trimethylphenoxy)-3-isopropylaminopropan-2-ol) (metipranolol);

(1-(isopropylamino)-3-(o-methoxyphenoxy)-3-[(1-methylethyl)amino]-2-propanol) (moprolol);

((1-tert.butylamino)-3-[(5,6,7,8-tetrahydro-cis-6,7-dihydroxy-1-naphthyl)oxy]-2-propanol) (nadolol);

((S)-1-(2-cyclopentylphenoxy)-3-[(1,1-dimethylethyl)-amino]-2-propanol sulfate (2:1)) (penbutolol);

(4'-[1-hydroxy-2-(amino)ethyl]methanesulfonanilide) (sotalol);

(2-methyl-3-[4-(2-hydroxy-3-tert.butylaminopropoxy)-phenyl]-7-methoxy-isoquinolin-1-(2H)-one);

(1-(4-(2-(4-fluorophenyloxy)ethoxy)phenoxy)-3-isopropylamino-2-propanol HCl);

((-)-p-[3-[(3,4-dimethoxyphenethyl)amino]-2-hydroxypropoxy]-ß-methylcinnamonitrile) (pacrinolol);

((±)-2-(3'-tert.butylamino-2'-hydroxypropylthio)-4-(5'-carbamoyl-2'-thienyl)thiazole HCl) (arotinolol);

((±)-1-[p-[2-(cyclopropylmethoxy)ethoxy]phenoxy]-3-(isopropylamino)-2-propanol) (cicloprolol);

((±)-1-[(3-chloro-2-methylindol-4-yl)oxy]-3-[(2-phenoxyethyl)amino]-2-propanol) (indopanolol);

((±)-6-[[2-[[3-(p-butoxyphenoxy)-2-hydroxypropyl]-amino]ethyl]amino]-1,3-dimethyluracil) (pirepolol);

(4-(cyclohexylamino)-1-(1-naphtholenyloxy)-2-butanol);

(1-phenyl-3-[2-[3-(2-cyanophenoxy)-2-hydroxypropyl]-aminoethyl]hydantoin HCl);

(3,4-dihydro-8-(2-hydroxy-3-isopropylaminopropoxy)-3-nitroxy-2H-1-benzopyran) (nipradolol);

α and ß-Adrenergic Blocking Agents:

((±)-1-tert-butylamino)-3-[o-[2-(3-methyl-5-isoxazolyl)vinyl]phenoxy]-2-propanol) (isoxaprolol);

(1-isopropylamino-3-(4-(2-nitroxyethoxy)phenoxy)-2-propanol HCl);

(4-hydroxy-α-[[3-(4-methoxyphenyl)-1-methylpropyl]-aminomethyl]-3-(methylsulfinyl)-benzmethanol HCl) (sulfinalol);

(5-[1-hydroxy-2-[[2-(o-methoxyphenoxy)ethyl]amino]-ethyl]-2-methylbenzenesulfonamide HCl);

(5-[1-hydroxy-2-[(1-methyl-3-phenylpropyl)amino]ethyl]-salicylamide HCl) (labetalol);

(1-((3-chloro-2-methyl-1H-indol-4-yl)oxy)-3-((2-phenoxyethyl)amino)-2-propanol-hydrogenmalonate) (ifendolol);

(4-(2-hydroxy-3-[(1-methyl-3-phenylpropyl)amino]-propoxy)benzeneacetamide);

(1-[3-[[3-(1-naphthoxy)-2-hydroxypropyl]-amino]-3,3-dimethyl-propyl]-2-benzimidazolinone);

(3-(1-(2-hydroxy-2-(4-chlorophenylethyl)-4-piperidyl)-3,4-dihydroxy)quinoxolin-2(1H)-one);

<u>CNS-Acting Agents</u>: clonidine; methyldopa;

<u>Adrenergic Neuron Blocking Agents</u>: guanethidine; reserpine and other rauwolfia alkaloids such as rescinnamine;

<u>Vasodilators</u>: diazoxide; hydralazine; minoxidil;

<u>Angiotensin I Converting Enzyme Inhibitors</u>:

1-(3-mercapto-2-methyl-1-oxopropyl)-L-proline (captopril);

(1-(4-ethoxycarbonyl-2,4(R,R)-dimethylbutanoyl)-indoline-2(S)-carboxylic acid);

(2-[2-[[1-(ethoxycarbonyl)-3-phenyl-propyl]amino]-1-oxopropyl]-1,2,3,4-tetrahydro-3-isoquinoline carboxylic acid);

((S)-1-[2-[[1-(ethoxycarbonyl)-3-phenylpropyl]amino]-1-oxopropyl]octahydro-1H-indole-2-carboxylic acid HCl);

(N-cyclopentyl-N-(3-(2,2-dimethyl-1-oxopropyl)thiol-2-methyl-1-oxopropyl)glycine) (pivalopril);

((2R,4R)-2-(2-hydroxyphenyl)-3-(3-mercaptopropionyl)-4-thiazolidinecarboxylic acid);
(1-(N-[1(S)-ethoxycarbonyl-3-phenylpropyl]-(S)-alanyl)-cis,syn-octahydroindol-2(S)-carboxylic acid HCl);
((-)-(S)-1-[(S)-3-mercapto-2-methyl-1-oxopropyl]-indoline-2-carboxylic acid);
([1(S),4S]-1-[3-(benzoylthio)-2-methyl-1-oxopropyl]-4-phenylthio-L-proline;
(3-([1-ethoxycarbonyl-3-phenyl-(1S)-propyl]amino)-2,3,4,5-tetrahydro-2-oxo-1-(3S)-benzazepine-1-acetic acid HCl);
(N-(2-benzyl-3-mercaptopropanoyl)-S-ethyl-L-cysteine) and the S-methyl analogue;
(N-(1(S)-ethoxycarbonyl-3-phenylpropyl)-L-alanyl-L-proline maleate) (enalapril);
N-[1-(S)-carboxy-3-phenylpropyl]-L-alanyl-1-proline;
$N^2$-[1-(S)-carboxy-3-phenylpropyl]-L-lysyl-L-proline (lysinopril);
2-[N-[(S)-1-ethoxycarbonyl-3-phenylpropyl]-L-alanyl-(1S,3S,5S)-2-azabicyclo[3.3.0]octane-3-carboxylic acid;
[1(S),4S]-1-(3-mercapto-2-methyl-1-oxopropyl)-4-phenylthio-L-proline S-benzoyl calcium salt (zofenopril);
[1-(+/-)4S]-4-cyclohexyl-1-[[2-methyl-1-[1-(1-(oxy-propoxy)propoxy](4-phenylbutyl)phosphinyl]acetyl]-L-proline sodium salt (fosfopril).

<u>Calcium Channel Blockers:</u> nifedepine; nitrendipine, verapamil; diltiazam.

Other Antihypertensive Agents: aminophylline; cryptenamine acetates and tannates; deserpidine; meremethoxylline procaine; pargyline; trimethaphan camsylate;

and the like, as well as admixtures and combinations thereof.

Typically, the individual daily dosages for these combinations can range from about one-fifth of the minimally recommended clinical dosages to the maximum recommended levels for the entities when they are given singly. Coadministration is most readily accomplished by combining the active ingredients into a suitable unit dosage form containing the proper dosages of each. Other methods of coadministration are, of course, possible.

The novel peptides of the present invention possess an excellent degree of activity in treating hypertension and congestive heart failure and they may be orally active.

For these purposes the compounds of the present invention may be administered orally, parenterally, by inhalation spray, or rectally in dosage unit formulations containing conventional non-toxic pharmaceutically acceptable carriers, adjuvants and vehicles. The term parenteral as used herein includes subcutaneous injections, intravenous, intramuscular, intrasternal injection or infusion techniques. In addition to the treatment of warm-blooded animals such as mice, rats, horses, dogs, cats, etc., the compounds of the invention are effective in the treatment of humans.

The pharmaceutical compositions may be in the form of a sterile injectable preparation, for example as a sterile injectable aqueous or oleagenous suspension. This suspension may be formulated according to the known art using suitable dispersing or wetting agents and suspending agents. The sterile injectable preparation may also be a sterile injectable solution or suspension in a non-toxic parenterally-acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium. For this purpose any bland fixed oil may be employed including synthetic mono- or diglycerides. In addition, fatty acids such as oleic acid find use in the preparation of injectables.

The peptides of this invention may also be administered in the form of suppositories for rectal administration of the drug. These compositions can be prepared by mixing the drug with a suitable non-irritating excipient which is solid at ordinary temperatures but liquid at the rectal temperature and will therefore melt in the rectum to release the drug. Such materials are cocoa butter and polyethylene glycols.

Dosage levels of the order of 0.1 to 4.0 grams per day parenterally are useful in the treatment of the above indicated conditions. Oral doses are 3-10 times higher. For example, renin-associated hypertension and hyperaldosteronism are

effectively treated parenterally by the administration of from 1.0 to 50 milligrams of the compound per kilogram of body weight per day.

The amount of active ingredient that may be combined with the carrier materials to produce a single dosage form will vary depending upon the host treated and the particular mode of administration.

It will be understood, however, that the specific dose level for any particular patient will depend upon a variety of factors including the activity of the specific compound employed, the age, body weight, general health, sex, diet, time of administration, route of administration, rate of excretion, drug combination and the severity of the particular disease undergoing therapy.

Thus, in accordance with the present invention there is further provided a pharmaceutical composition for treating hypertension and congestive heart failure, comprising a pharmaceutical carrier and a therapeutically effective amount of a peptide of the formula I.

Also, in accordance with the present invention there is still further provided a method of treating hypertension and congestive heart failure, comprising administering to a patient in need of such treatment, a therapeutically effective amount of a peptide of the formula I.

The renin inhibitory peptides of the present invention may also be utilized in diagnostic methods for the purpose of establishing the significance of renin as a causative or contributory factor in hypertension or congestive heart failure in a

particular patient. For this purpose the present peptides may be administered in a single dose of from 0.1 to 10 mg per kg of body weight.

Both in vivo and in vitro methods may be employed. In the in vivo method, a novel peptide of the present invention is administered to a patient, preferably by intravenous injection, although other routes of parenteral administration are also suitable, at a hypotensive dosage level and as a single dose, and there may result a transitory fall in blood pressure. This fall in blood pressure, if it occurs, indicates supranormal plasma renin levels.

Some of the peptides of formula I also have angiotensin converting enzyme (ACE) inhibitor activity. This activity augments renin inhibition in lowering blood pressure and in treating congestive heart failure.

The compounds of the present invention, characterized by formula I may be viewed as a peptide segment A-B-D linked through an amide bond to a phosphorus-containing component designated as E. The preparation of these compounds is illustrated in the examples below, and in general proceeds as follows:

1. The phosphorus-containing unit E is prepared as outlined in the schemes below. In general, the unit G is incorporated into an intermediate E-G during or after this process. Then an amino protecting group is removed from E-G.
2. The unit A-B-D is coupled to E-G in one of the following ways:
   a) Coupling of an amino-protected form of D to E-G, followed by amino protecting group

removal from D-E-G and coupling of A-B to the deprotected amino group of D-E-G.

b) Coupling of A-B-D, prepared by known techniques as illustrated in the examples to follow, to E-G by known coupling procedures.

The units E and G may contain functionality which requires protection during synthesis of E and coupling of E to G. Protecting groups, among those well-known in peptide synthesis, are chosen so as to be compatable with these steps and so as to allow selective deprotection of the amino function in the unit E, which is then to be coupled to the A-B-D portion of the peptide.

The phosphorus-containing component E, as well as the amino acid side-chains of B and D may likewise contain functionality which requires protection during the subsequent coupling reactions. Again, protecting groups are chosen so as to be compatable with the coupling steps, yet easily removable afterwards. In general, the carbobenzoxy group (CBZ) is used to protect the amino function in E which becomes coupled to the A-B-D unit. Other amino groups are protected with t-butoxycarbonyl (BOC), 9-fluorenylmethyloxycarbonyl (FMOC) and benzyl (BZ) groups. Carboxylic acids are protected as the methyl, ethyl, benzyl, or t-butyl esters. Phosphonic acids are protected as the methyl, ethyl, t-butyl, or benzyl esters. The peptide couping procedures referred to above include those brought about by use of dicyclohexylcarbodiimide/1-hydroxybenzotriazole and of disuccinimido oxallate (K. Takeda et al, Tetrahedron Lett., 24, 4451-54 (1983)). When the B

or D units are N-methylamino acids, or when the amino group of the unit E which is to be coupled to A-B-D bears an N-methyl group ($R^6$=$-CH_3$), procedures well known in peptide synthesis are used to prepare the A-B-D segments and to couple this unit to E or E-G. In general, the mixed anhydride procedure (with pivaloyl chloride and N-methylmorpholine) is used, as illustrated by R. M. Wenger, Helv. Chem. Acta., 1984, 67, 502-525. N-methylamino acids can be prepared by the method of M. J. O'Donnell et al., Tetrahedron Lett., 1984, 25, 3651.

Preparation of the phosphorus-containing components E are carried out as illustrated in the examples to follow.

The 1-aminoalkylphosphonous acids used as starting materials in the examples below are prepared as illustrated in the examples and can be resolved to give optically active materials by the method of Baylis et al. (J. Chem. Soc. Perkin Trans 1, 2845-53 (1984)). Compounds derived from both the optically active and racemic materials are claimed in the present invention.

The 1-aminoalkylphosphonic acids used in the examples below are prepared as described and can be resolved to give the optically active materials by the procedure of Kafarski et al, Can. J. Chem., 60, 3081-84 (1982), or can be prepared in optically active form by the method of Huber et al, Tetrahedron Lett. 3049-52 (1979). Compounds derived from both the optically active and racemic materials are claimed in the present invention.

The following schemes and examples illustrate the preparation of peptides of formula I. In these schemes and examples, the following abbreviations are used: DPPA = diphenylphosphorylazide; DCC = dicyclohexylcarbodiimide; HOBT = 1-hydroxybenzotriazole hydrate; TFA = trifluoroacetic acid; CBZ = carbobenzoxy; BOC = t-butoxycarbonyl; DNP = 2,4-dinitrophenyl; FMOC = 9-fluorenylmethyloxycarbonyl; Bz = benzyl; HOAc = acetic acid; TMS-Cl = trimethylsilyl chloride; AIBN = azobis isobutyronitrile.

It will be understood that the following schemes outline representative examples of the preparation of peptides of formula I and that similar peptides possessing alternative substituents can equally well be prepared by the routes outlined.

The following schemes illustrate the preparation of E units of formulas VII, VIII, IX, X, and XI (in protected form and their incorporation into peptide of formula I:

1.

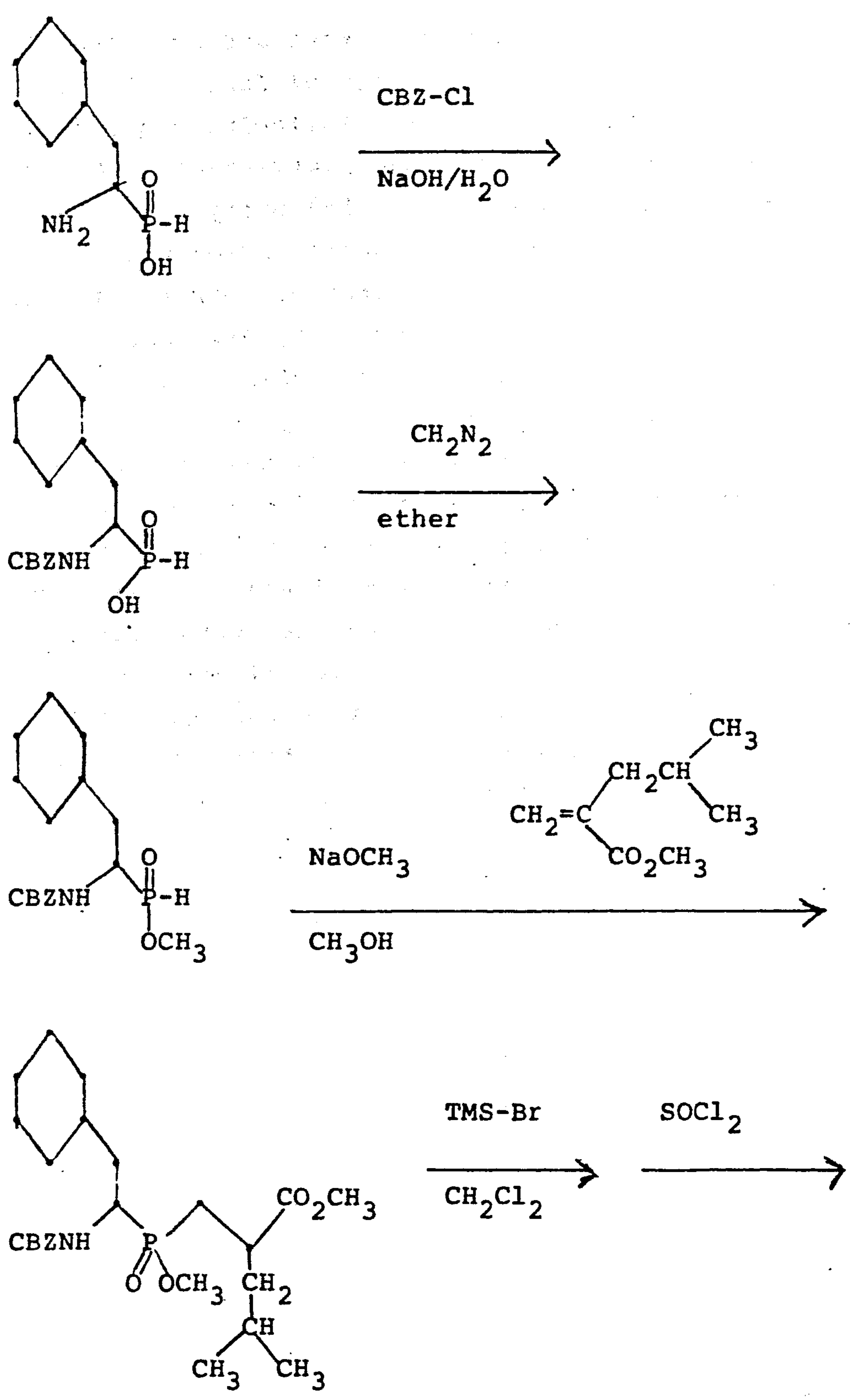


XII

XIII

XIV

XV

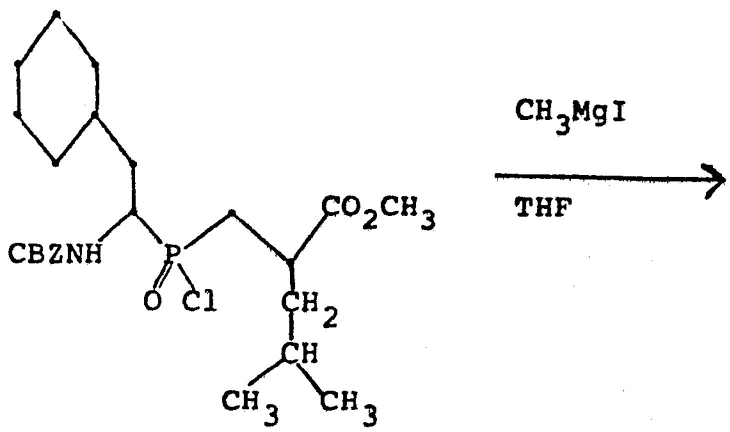


XVI

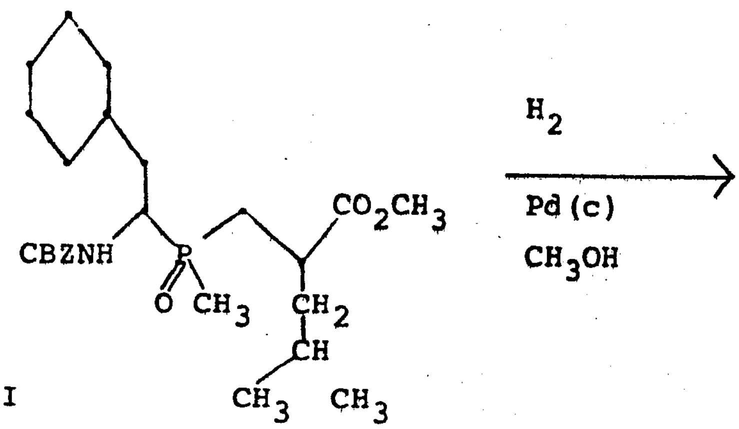


XVII

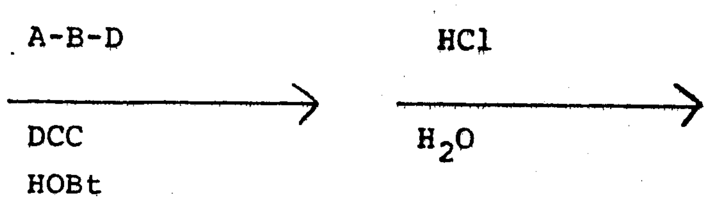


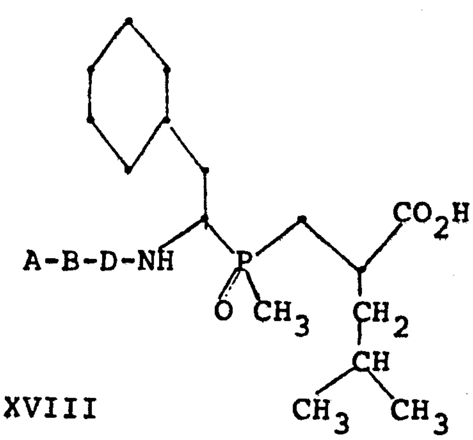

XVIII

2.

XIII

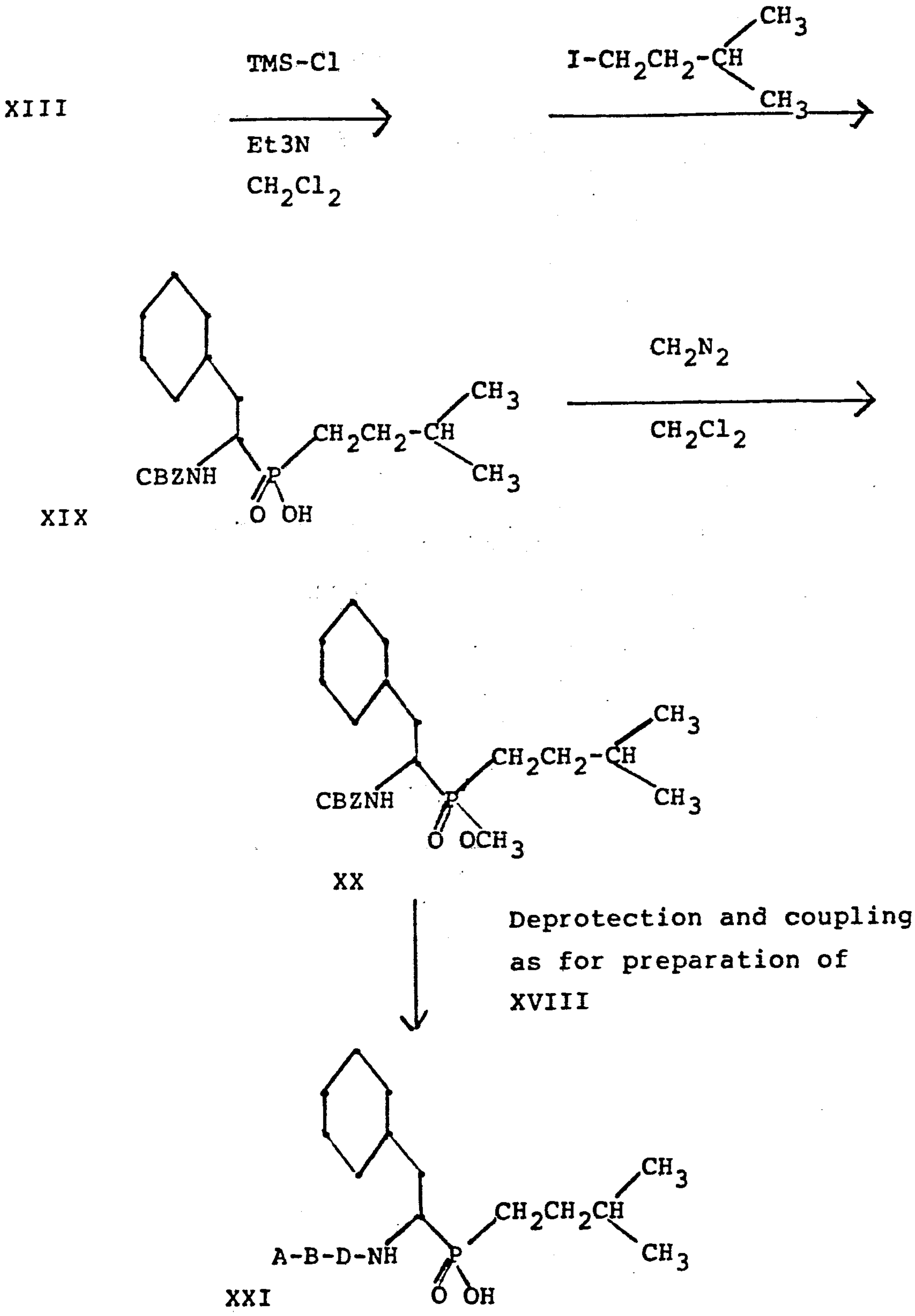


XIX

XX

XXI

3.

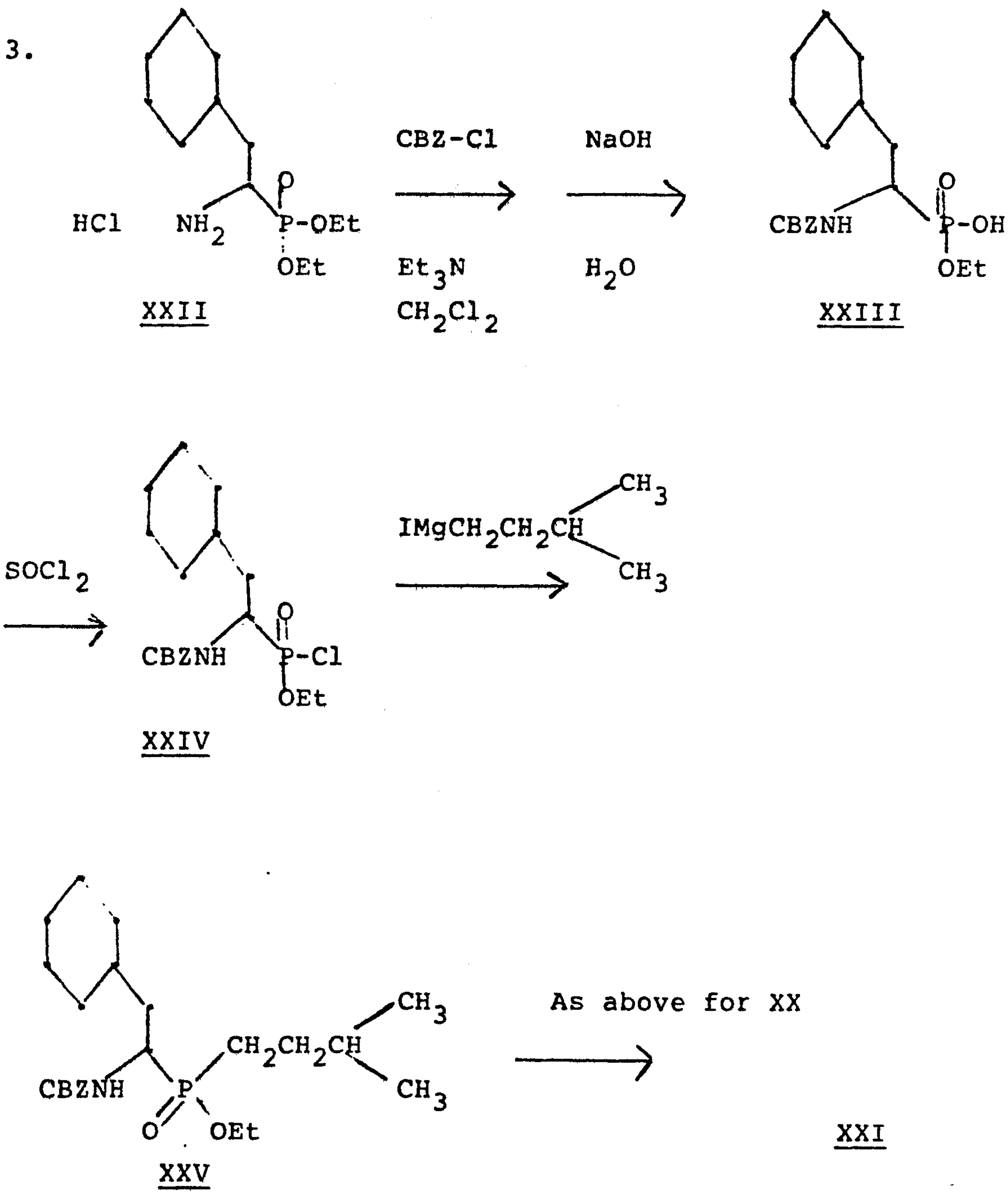


XXII

XXIII

XXIV

XXV

XXI

4.

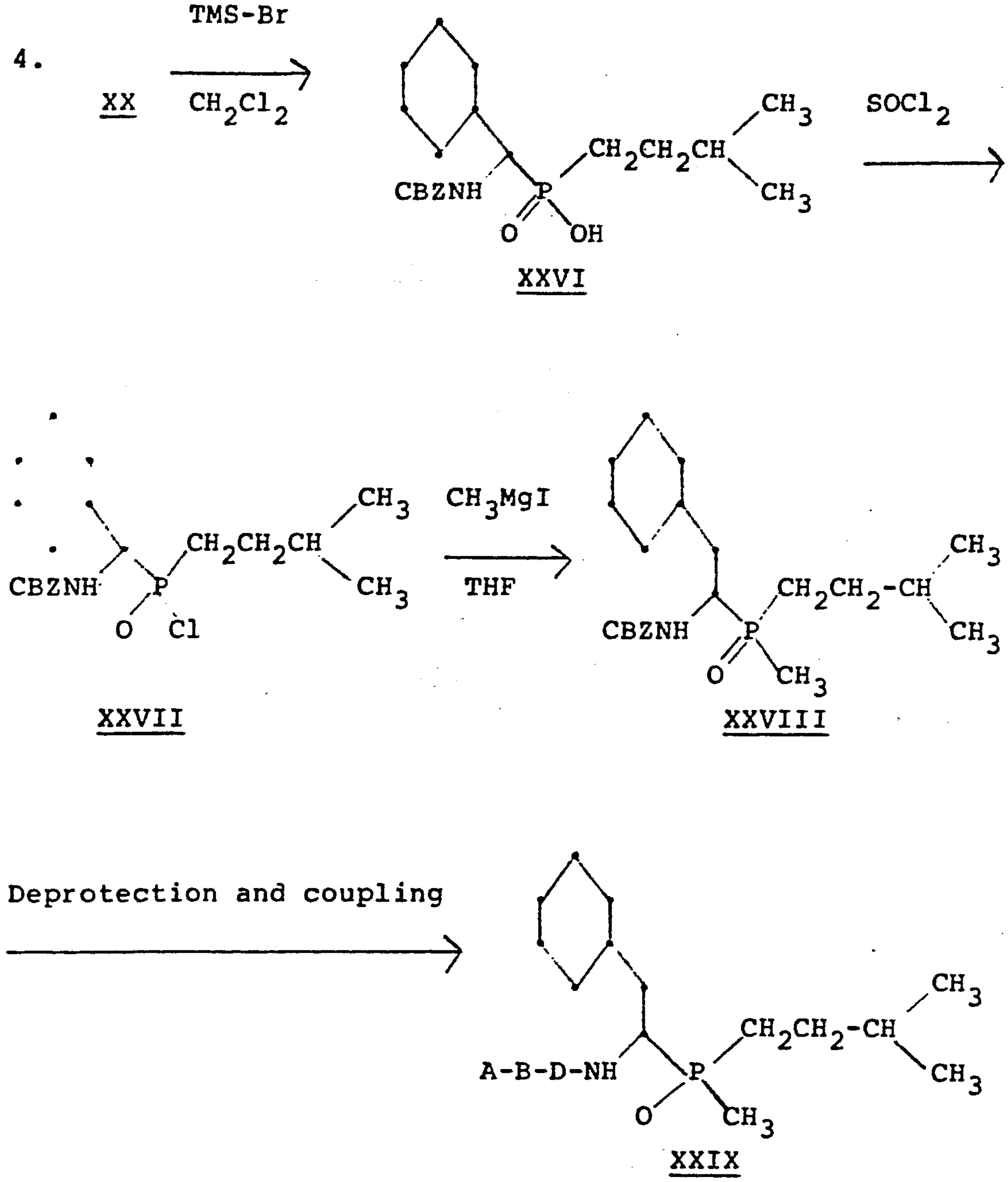
XX
TMS-Br
$CH_2Cl_2$
XXVI
$SOCl_2$
XXVII
$CH_3MgI$
THF
XXVIII
Deprotection and coupling
XXIX

5.

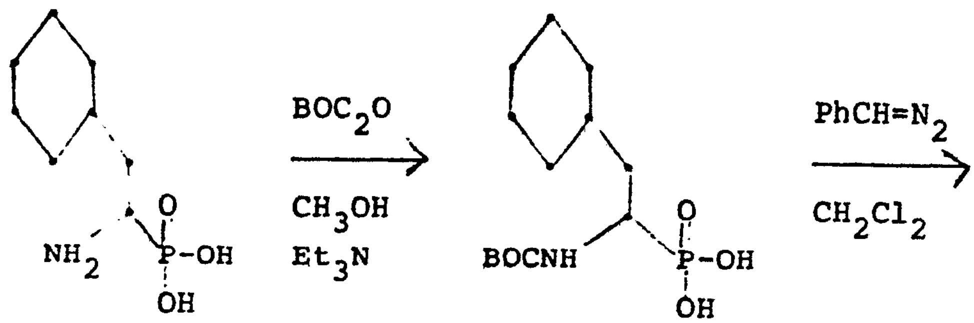


XXX XXXI

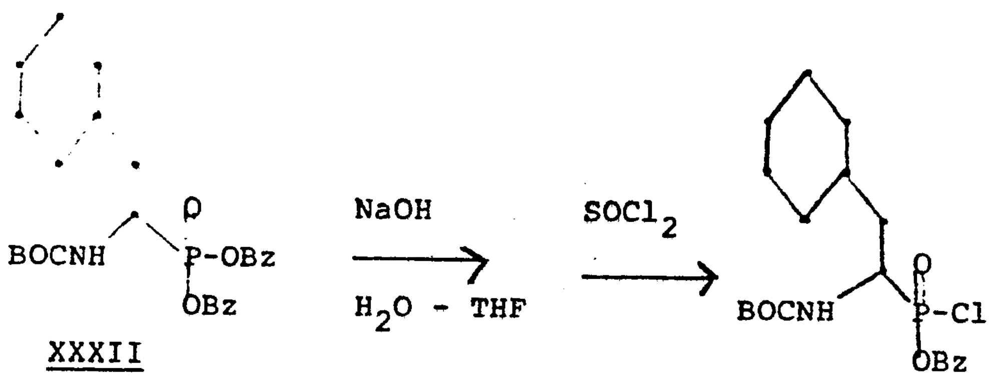


XXXII XXXIII

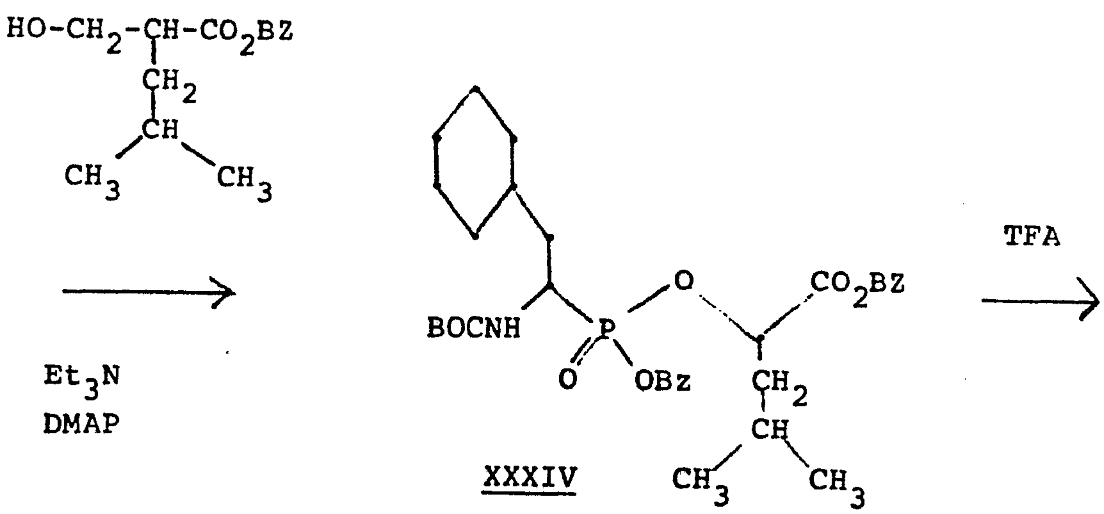


XXXIV

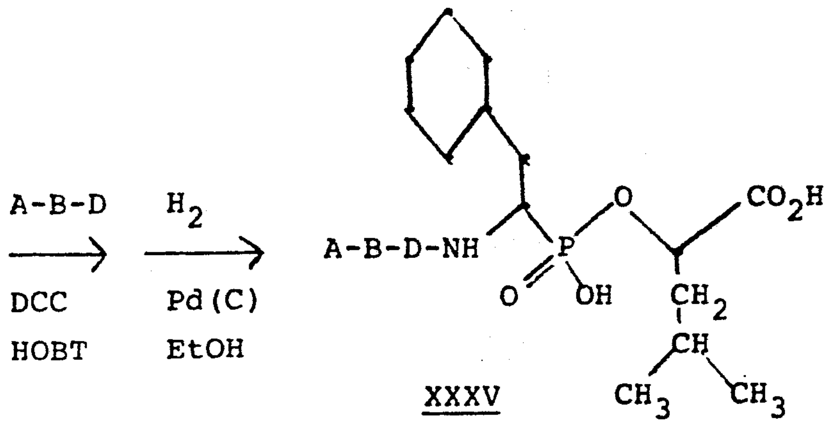


XXXV

6.

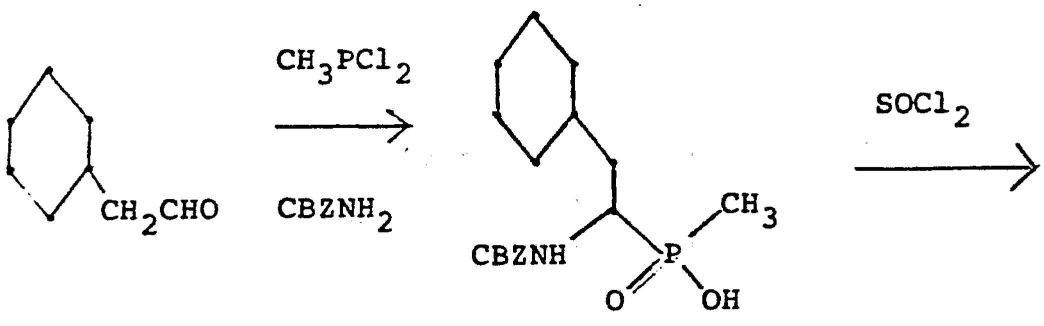


XXXVI

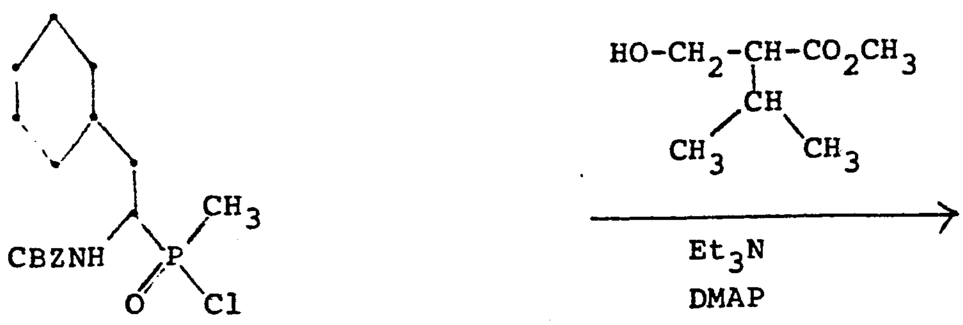


XXXVII

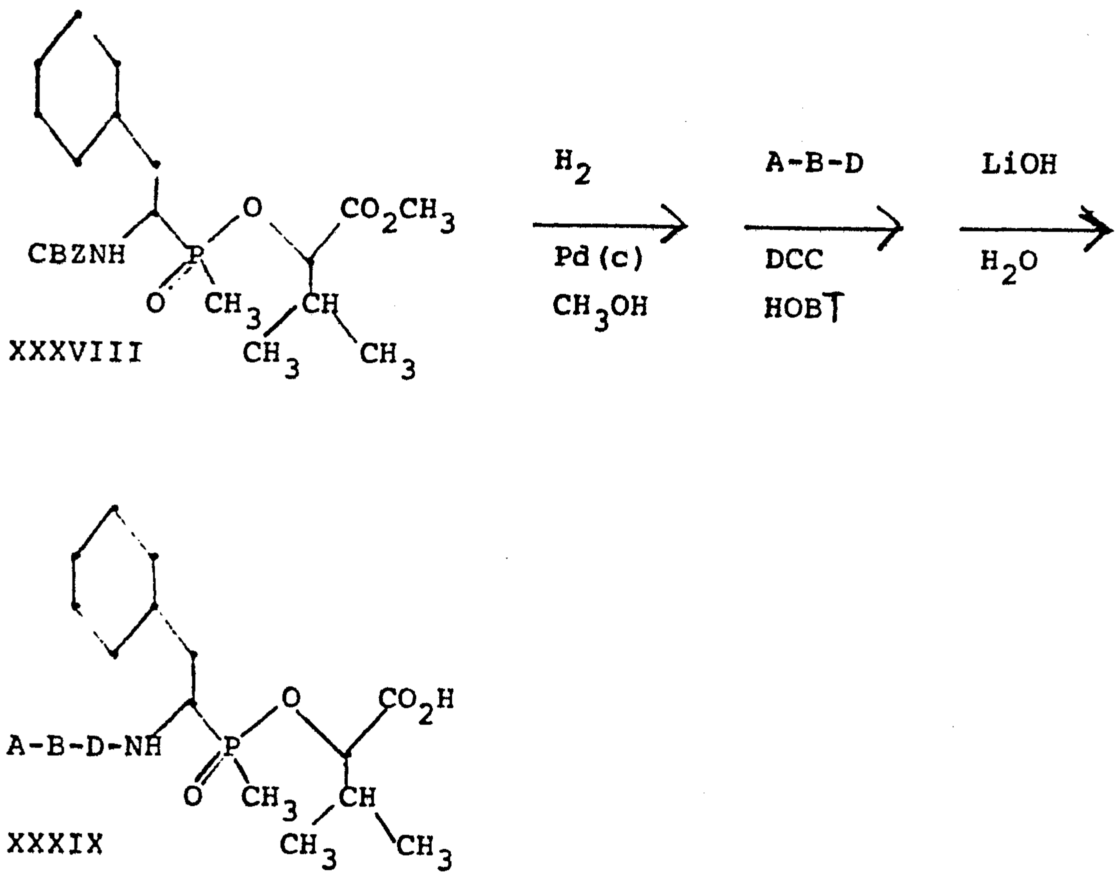


XXXVIII

XXXIX

7.

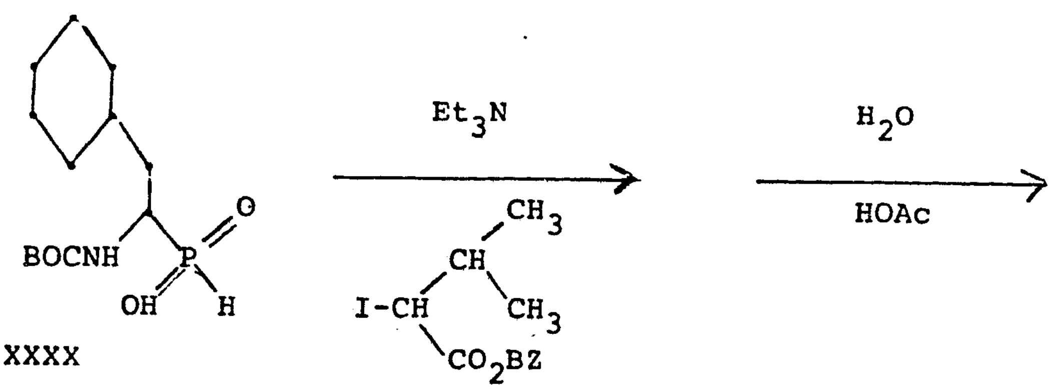


XXXX

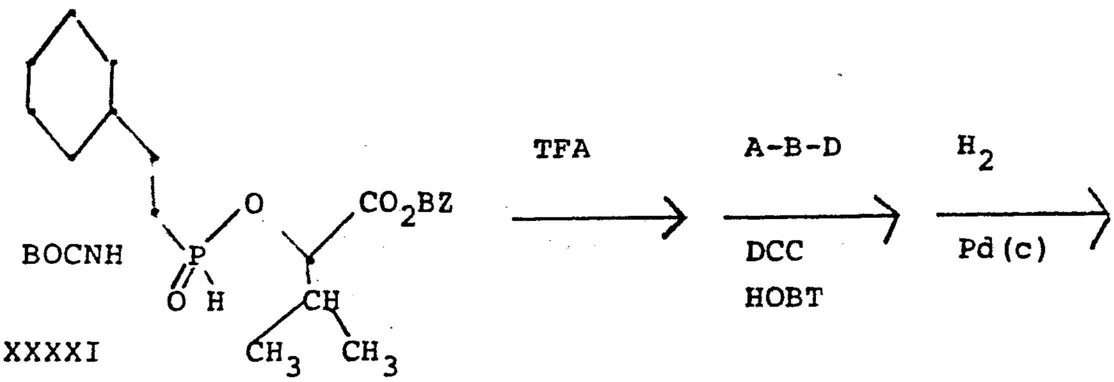


XXXXI

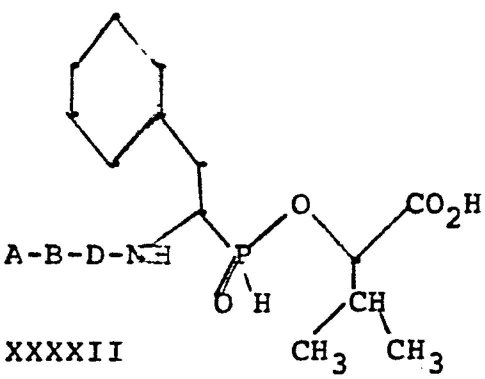

XXXXII

8. XIII

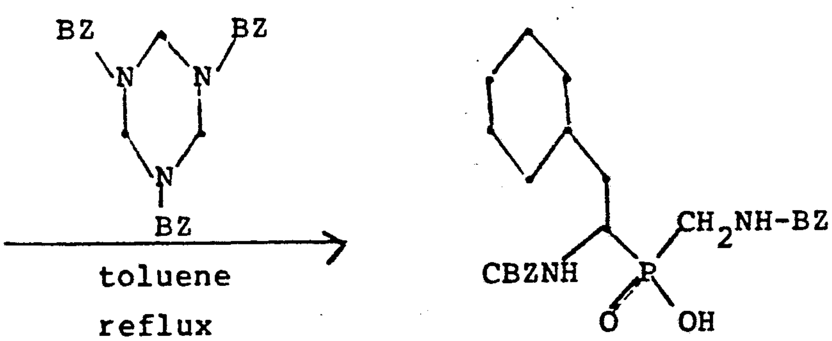


XXXXIII

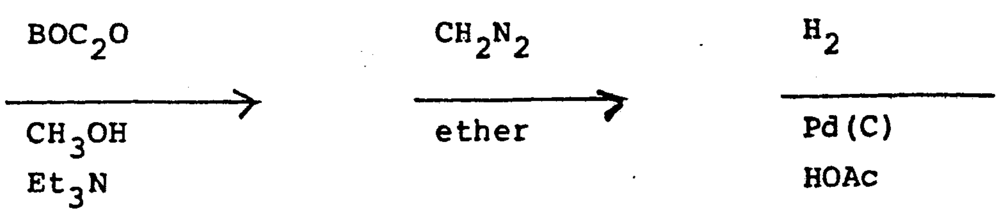

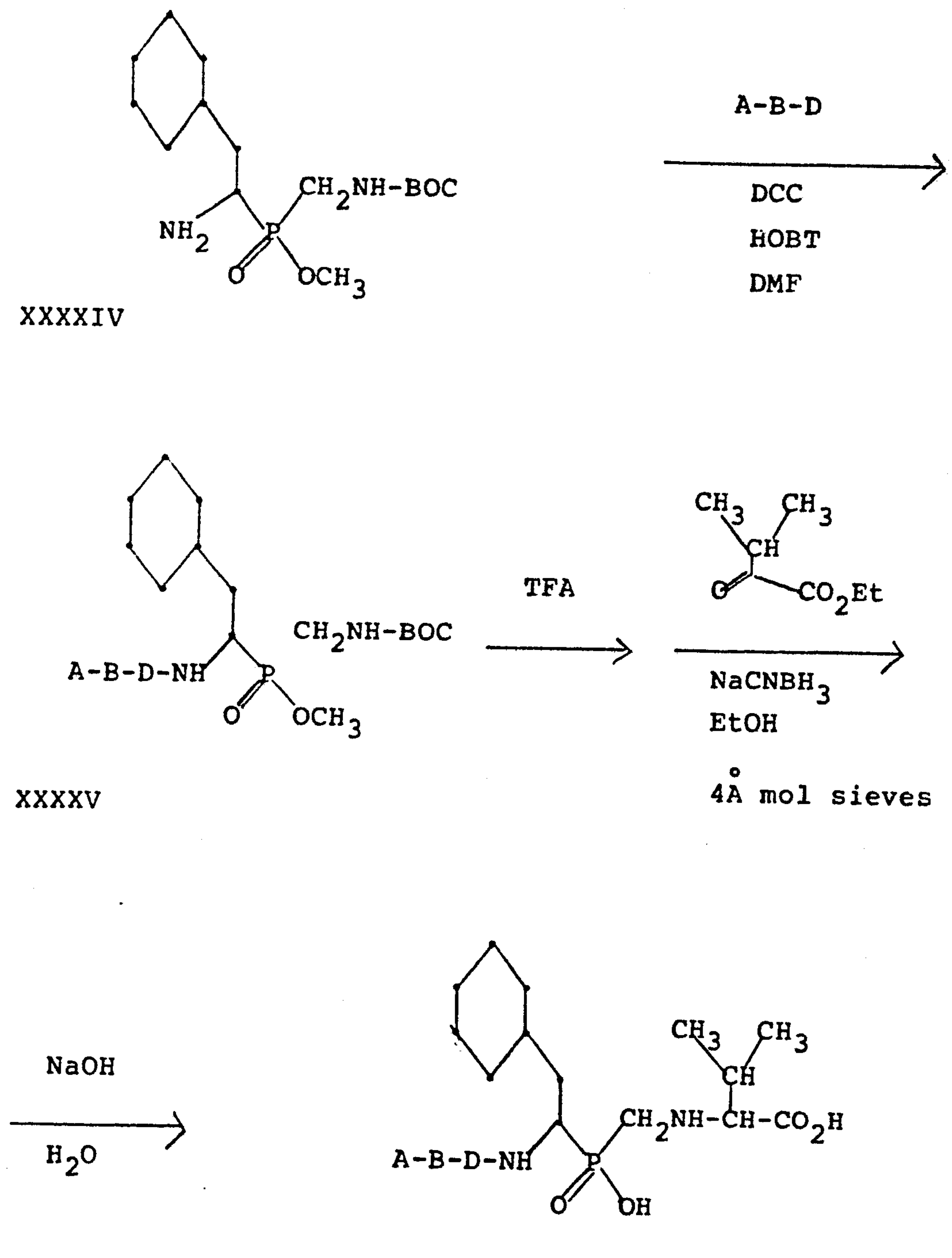


XXXXIV

XXXXV

XXXXVI

The component E may also be a phosphinate ester of formula XXXXVII:

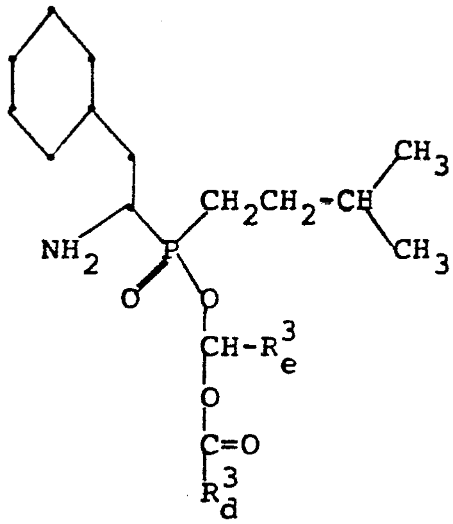

Peptide XXXXIX containing XXXXVII may be prepared as illustrated in the following scheme:

XIX

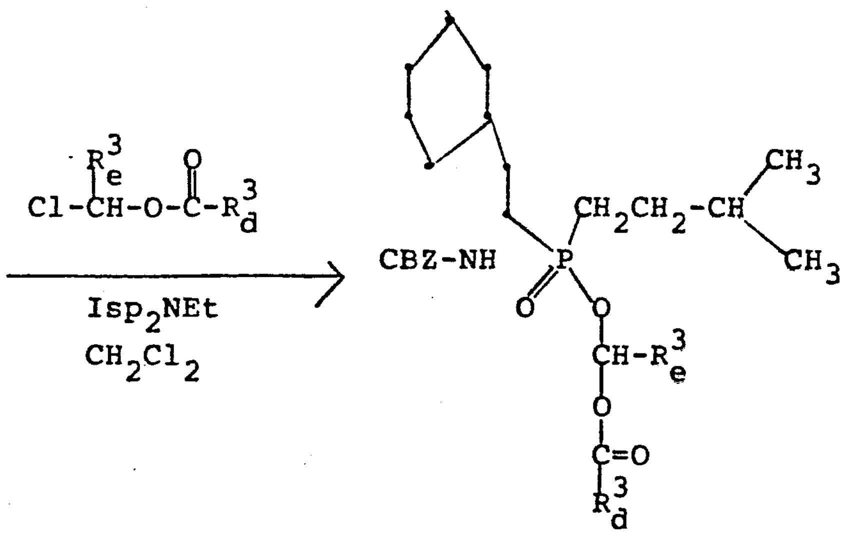


XXXXVIII

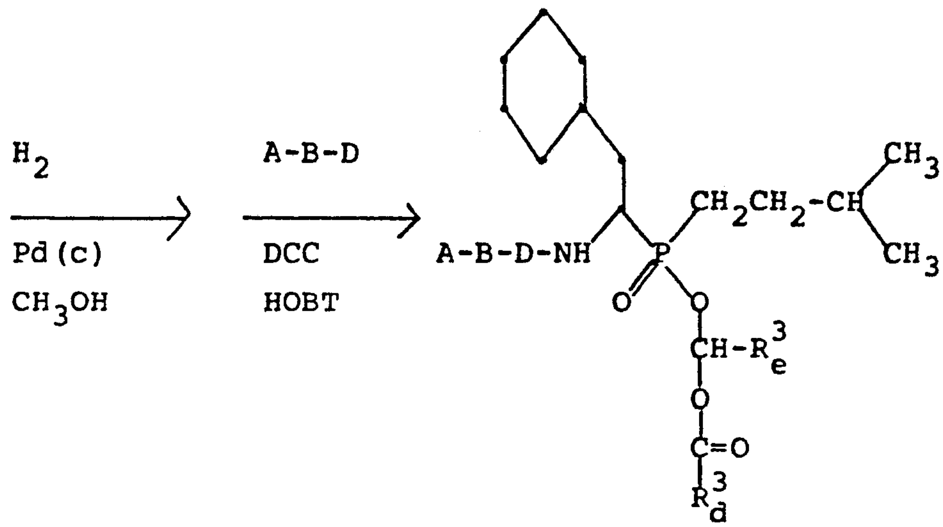


XXXXIX

Components L may be prepared as illustrated below:

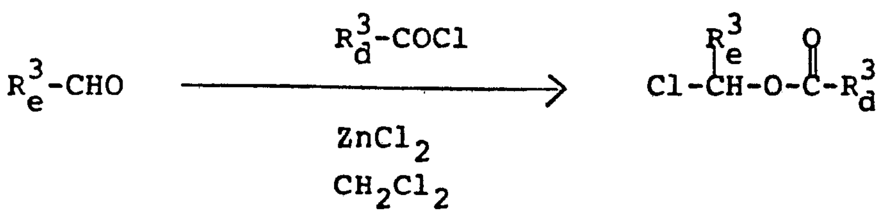


The following examples illustrate preparation of intermediates and products of the present invention. Melting points were recorded on a Thomas-Hoover melting point apparatus and are uncorrected as are all boiling points. $^1H$ NMR spectra were taken on a Varian XL-300 FT spectrometer. Chemical shifts are reported in ppm downfield from tetramethylsilane as internal standard. IR spectra were recorded on a Perkin-Elmer Model 297 spectrometer. Optical rotations were measured with a Perkin Elmer 141 automatic

polarimeter in the solvents indicated. Mass spectra (MS) were taken on a Varian 731 spectrometer at 70 eV. Those marked FAB were taken by using the fast atom bombardment method.

The following examples illustrate preparation of compounds of the present invention. Temperatures are in °C unless otherwise indicated.

## EXAMPLE 1

Cyclohexylacetaldehyde

A suspension of 100 g (0.46 moles) of pyridinium chlorochromate and 100 g of celite in 800 ml of methylene chloride was stirred vigorously while 38 g (0.3 moles) of 2-cyclohexylethanol in 200 ml of methylene chloride was added all at once. The reaction turned dark immediately and became mildly exothermic. After 1 hour, 1000 ml of ether was added and the reaction mixture was filtered through a bed of silica gel (ca. 250 g) on a fritted glass disk. The pad was rinsed with an additional liter of ether. The combined filtrates were reduced in volume to approximately 200 ml and the solution was washed with 2 x 40 ml of 6N HCl, 1 x 50 ml of saturated sodium bicarbonate, and 1 x 50 ml of saturated NaCl solution. The organic layer was dried over magnesium sulfate, filtered and evaporated *in vacuo* to give a light green oil. The residue was distilled *in vacuo* to afford 21 g (56%) of a colorless oil (bp 74-76°C at 23 mm of Hg). NMR ($CDCl_3$) (60 MHz): 0.8-2.1 (m, 11H); 2.2-2.4 (m, 2H); 9.6 (t, J=2 Hz, 1H) ppm.

EXAMPLE 2

1-Amino-2-cyclohexylethylphosphinic acid

A stirred slurry of 26.00 g (0.118 moles) of aminodiphenylmethane-HCl in 100 ml of absolute ethanol was treated with 15.50 g (0.123 moles) of cyclohexylacetaldehyde, immediately followed by 12.8 ml (0.123 moles) of hypophosphorus acid (50% aqueous). The reaction mixture was heated in an oil bath held at 100°C. As the reaction approached reflux it became homogeneous, then heterogeneous again after approximately 5 minutes (white precipitate). After 45 minutes at reflux, an additional 100 ml of ethanol was added to the very thick slurry. Reflux was continued for an additional 3 hours 15 minutes. At this time the reaction mixture was cooled to 0°C in an ice bath, then the solid filtered off. The white solid was washed with 50 ml of ice-cold ethanol and air dried.

The white solid was added to 150 ml of glacial acetic acid and 150 ml of 48% aqueous HBr added. Dissolution occurred over a period of 5 minutes, and the reaction turned a light yellow color. After another 10 minutes a white solid precipitated out of solution. Stirring at room temperature was continued for a total of 2 hours. The flask was then immersed in an oil bath preheated to 115°C. After 1 hour the reaction was almost homogeneous. After a total of 3 hours at 115°C, the reaction mixture was cooled to 0°C in an ice bath. The solution was washed with 1 x 200 ml and 2 x 100 ml of hexanes. The hexanes wash was discarded and the remaining aqueous acid solution was evaporated to

dryness on a rotary evaporator. The resulting semi-crystalline foam was dissolved in 125 ml of absolute ethanol and cooled to 0°C in an ice bath. Propylene oxide (50 ml) was slowly added and a white precipitate was formed. The reaction mixture was allowed to warm to room temperature of its own accord while stirring. After a total of 18 hours, the slurry was cooled to 0°C again, and the solid filtered off. The solid was washed with 100 ml more of ice-cold ethanol and dried to afford 11.98 g (53% overall) of a white solid mp 220-221°C (turns orange and bubbles). NMR ($D_2O$) (60 MHz): 0.8-2.1 (m, 14H); 3.3 (m, 2H); 7.0 (d, J=527 Hz, 1H) ppm.

## EXAMPLE 3

Methyl N-CBZ-1-Amino-2-cyclohexylethylphosphinate

A solution of 7.00 g (0.037 moles) of 1-amino-2-cyclohexylethylphosphonous acid in 105 ml of dioxane and 40 ml of 1N NaOH was cooled to 0°C in an ice bath and stirred vigorously while 10.50 ml (12.53 g; 0.073 moles) of benzyl chloroformate and 80 ml of 1N NaOH were added rapidly and simultaneously over a period of approximately 1 minute. The pH was adjusted to the 8-9 range using 1N NaOH (hydrion paper) added in small increments. The reaction mixture was allowed to come to room temperature, and vigorous stirring was continued for 48 hours. The dioxane was removed *in vacuo* and the aqueous washed with 100 ml of ether. The ether layer was discarded and the aqueous solution acidified using 1N $KHSO_4$ to approximately pH = 1-2 (hydrion paper). The solution was extracted with 5 x 100 ml of ethyl

acetate. The combined ethyl acetate layers were dried over anhydrous $Na_2SO_4$, filtered, and evaporated in vacuo to afford N-CBZ-1-amino-2-cyclohexylethylphosphonous acid as a white solid.

The solid was redissolved in 200 ml of ethyl acetate and 150 ml of ethereal diazomethane was added all at once. The reaction was stirred at room temperature for 1 hour, at which time the volatiles were removed completely in vacuo to give a viscous oil. This material was crystallized using 200 ml of 1:1 ether:petroleum ether to afford 3.45 g of product. The mother liquors obtained after evaporation of the filtrate were chromatographed on silica gel using 18:1:1 methylene chloride:acetone:methanol to give an additional 5.69 g of product. Total product = 9.14 g (73%). NMR ($CDCl_3$) (60 MHz): 0.8-2.2 (m, 14H); 3.6, 3.8 (s, 3H); 3.8-4.6 (br, 1H); 5.2 (s, 2H); 6.9 (d, J=542 Hz, 1H); 7.2 (s, 5H) ppm.

## EXAMPLE 4

### Methyl N-BOC-1-amino-2-cyclohexylethylphosphinate

A solution of 0.955 g (0.005 moles) of 1-amino-2-cyclohexylethylphosphonous acid in 10 ml of dioxane and 10 ml of 0.5N NaOH was cooled to 0°C in an ice bath. The reaction mixture was vigorously stirred while 1.26 ml (1.20 g; 0.0055 moles) of di-tert-butyldicarbonate was added all at once. The reaction mixture was allowed to come to room temperature. After a total reaction time of 17 hours, the dioxane was removed in vacuo and the aqueous was acidified with 1N $KHSO_4$. The mixture was extracted with 3 x 50 ml of ethyl acetate. The

combined ethyl acetate solution was dried (anhydrous $Na_2SO_4$) and evaporated *in vacuo* to a slightly cloudy oil.

The oil was redissolved in 50 ml of ethyl acetate and treated with 50 ml of ethereal diazomethane solution. The reaction mixture was stirred at room temperature for 2 hours, then the volatiles were removed completely *in vacuo* to give a thick oil. The crude product was chromatographed on silica gel using 18:1:1 methylene chloride:acetone:methanol as the eluant to give 1.17 g (100%) of product as a very thick oil. NMR ($CDCl_3$) (60 MHz): 0.0-2.2 (m containing 9H s at 1.5, 23H (total)); 3.7, 3.9 (s, 3H); 5.2-6.0 (m, 1H); 6.9 (d, J=552 Hz, 1H) ppm.

## EXAMPLE 5

Methyl (N-CBZ-1-amino-2-cyclohexylethyl) 2-carbomethoxy-4-methylpentylphosphinate

A solution of 2.75 g (0.008 moles) of methyl 1-CBZ-amino-2-cyclohexylethylphosphinate in 25 ml of absolute methanol was cooled to 0°C in an ice bath and 4.60 ml of 2M NaOMe in methanol (0.009 moles) was added *via* syringe. The reaction mixture was stirred at 0°C for 10 minutes, at which time 1.21 g (0.009 moles) of methyl 2-(2-methylpropyl)acrylate was added all at once. The reaction was allowed to proceed at 0°C for 30 minutes, then the ice bath was removed and the reaction was allowed to proceed at room temperature for 18 hours. The methanol was removed *in vacuo* and the residue treated with 100 ml of 1N HCl. The aqueous was extracted with 3 x 50 ml of ethyl acetate. The combined ethyl acetate was dried over

anhydrous $MgSO_4$, filtered, and evaporated in vacuo to give a colorless oil. The crude product was chromatographed on silica gel using 18:1:1 methylene chloride:acetone:methanol as the eluant to afford 1.91 g (49%) of the product as a white solid. NMR ($CDCl_3$) (60 MHz): 0.8-2.2 (m, 14H); 3.6, 3.8 (s, 3H); 5.1 (s, 2H); 7.0 (d, J=535 Hz, 1H); 7.3 (s, 5H) ppm.

## EXAMPLE 6

Methyl (N-BOC-1-amino-2-cyclohexylethyl) 2-carbomethoxy-4-methylpentylphosphinate

A solution of 4.85 g (0.021 moles) of the ester from Example 4 in 35 ml of absolute methanol was cooled to 0°C, whereupon 11.45 ml of 2N NaOMe in methanol (0.023 moles) was added via syringe. The reaction was stirred for 10 minutes, at which time 3.10 g (0.022 moles) of methyl 2-(2-methylpropyl)-acrylate was added all at once. Stirring was continued at 0°C for 30 minutes, then the ice bath was removed and stirring was continued at room temperature for 19 hours. At that time the methanol was removed in vacuo and the residue treated with 200 ml of 1N HCl. The aqueous was extracted with 3 x 50 ml of ethyl acetate. The combined ethyl acetate washes were dried ($MgSO_4$), filtered and the volatiles evaporated in vacuo to give an oil. The crude product was purified by silica gel chromatography using ethyl acetate as an eluant to afford 4.77 g (61%) of the product as a very viscous oil. NMR ($CDCl_3$) (60 MHz): 0.9 (m, 6H); 0.8-2.2 (m, containing 9H s at 1.5, 29H total); 3.6, 3.8 (s, 3H); 3.7 (s, 3H) ppm.

## EXAMPLE 7

Methyl (1-amino-2-cyclohexylethyl) 2-carbomethoxy-4-methylpentylphosphinate

A mixture of 1.86 g (0.004 moles) of the ester product of Example 5 and 0.95 g of 10% Pd on carbon in 30 ml of absolute methanol was hydrogenated on a Parr type apparatus at 40 psig of hydrogen for 20 hours. The reaction mixture was filtered through a small pad of celite and the pad washed well with methanol. The filtrate was evaporated completely *in vacuo* to afford the pure free amine (1.34 g; 100%) as a viscous oil. NMR ($CDCl_3$) (300 MHz) 0.8-1.0 (m, 6H); 0.8-3.0 (m, 20H); 3.7-4.0 (series of s, total 6H); 8.1 (very br s, 2H) ppm.

## EXAMPLE 8

Methyl (1-amino-2-cyclohexylethyl) 2-carbomethoxy-4-methylpentylphosphinate hydrochloride

A solution of 1.13 g (0.003 moles) of the product of Example 6 in 20 ml of absolute methanol was treated with 12 ml of HCl/methanol (144 g of HCl in 400 ml methanol). The reaction mixture was stirred at room temperature for 4.5 hours. Analysis by thin layer chromatography indicated that no more starting material remained. The volatiles were removed *in vacuo* and replaced several times with more methanol and re-evaporated. After vacuum drying, this affrorded 1.07 g (100%) of the product as a glassy froam. NMR ($CDCl_3$) (60 MHz): 0.9 (m, 6H); 0.8-3.0 (m, 20H); 3.5-4.1 (m, 6H); 8.6 (very br s, 3H) ppm.

EXAMPLE 9

N-CBZ-2-Amino-3-(1-naphthyl)propionic acid

A solution of 0.700 g (0.003 moles) of 2-amino-3-(1-naphthyl)propionic acid in 10 ml of dioxane and 3.2 ml of 1N NaOH was cooled to 0°C in an ice bath and 6.40 ml of 1N NaOH and 0.923 ml (1.10 g; 0.0064 moles) of benzyl chloroformate were added simultaneously and rapidly *via* syringe. The reaction mixture was stirred vigorously and the pH was adjusted to the 8-9 range by adding 1N NaOH dropwise. The reaction was allowed to come to room temperature of its own accord while stirring vigorously. After 24 hours at room temperature, the dioxane was removed *in vacuo* and the aqueous washed with 2 x 10 ml of ether. The ether layers were discarded and the aqueous layer was acidified to pH=1-2 (hydrion paper) using 1N $KHSO_4$. The aqueous solution was extracted with 2 x 50 ml of ethyl acetate. The combined ethyl acetate washings were dried over anhydrous $MgSO_4$, filtered, and the volatiles removed *in vacuo* to give a clear oil. This was triturated with 5 x 5 ml portions of petroleum ether and dried *in vacuo* to afford 1.09 g (97%) of the product. NMR ($CDCl_3$) (300 MHz): 3.4 (dd, 1H); 3.8 (dd, 1H); 4.8 (dd, 1H); 5.0 (s, 2H); 5.3 (d, 1H); 7.1-7.6 (m, 9H); 7.8 (d, 1H); 7.9 (d, 1H); 8.1 (d, 1H) ppm.

EXAMPLE 10

N-BOC-2-Amino-3-(1-naphthyl)propionic acid

A solution of 0.700 g (0.003 moles) of 2-amino-3-(1'-naphthyl)propionic acid in 7 ml of dioxane and 13 ml of 0.5 N NaOH was cooled to 0°C in

an ice bath and stirred vigorously while 0.82 ml (0.773 g; 0.0036 moles) of di-tert-butyl dicarbonate was added in one portion. The ice bath was removed and the heterogeneous reaction mixture was stirred vigorously at room temperature. After several hours, the reaction mixture became homogeneous. Vigorous stirring was continued for ca. 24 hours. The dioxane was removed *in vacuo* and the residue acidified to pH=1-2 with 1N $KHSO_4$. The aqueous was extracted with 2 x 50 ml of ethyl acetate. The combined organic extracts were dried over anhydrous $MgSO_4$, filtered, and the volatiles evaporated *in vacuo* to give a colorless oil. The oil was triturated 10 ml of petroleum ether to give 0.97 g (94%) of the product as a white powder (crystallization occurs very slowly). NMR ($CDCl_3$) (300 MHz): 0.7 (s, 9H); 3.2 (dd, 1H); 3.9 (dd, 1H); 4.7 (m, 1H); 7.3-7.6 (m, 5H); 7.8 (d, 1H); 7.9 (d, 1H); 8.2 (d, 1H) ppm.

## EXAMPLE 11

Methyl 2-(2-methylpropyl)acrylate

This compound was prepared by the method of J. Harley-Mason *et al*. (*Tetrahedron* 1980, 36, 1063) in approximately 35% overall yield. NMR ($CDCl_3$) (300 MHz): 0.9 (d, 6H); 1.8 (septet, 1H); 2.2 (d, 2H); 3.7 (s, 3H); 5.5 (d, 1H); 6.1 (d, 1H) ppm.

## EXAMPLE 12

Methyl 2-(cyclohexylmethyl)acrylate

This compound was prepared as above in approximately 20% overall yield from dimethyl

malonate and bromomethylcyclohexane. NMR ($CDCl_3$) (60 MHz): 0.8-2.0 (m, 11H); 2.2 (d, 2H); 3.7 (s, 3H); 5.4 (m, 1H); 6.0 (d, J=2Hz, 1H) ppm.

EXAMPLE 13

Methyl 2-(n-propyl)acrylate

This compound was prepared as above in approximately 45% overall yield from dimethyl malonate and n-propyl bromide. bp.=51-53°C at 20 mm of Hg. NMR ($CDCl_3$) (60 MHz): 0-.9 (t, J=7Hz, 3H); 1.2-1.8 (m, 2H); 2.3 (t, J=7Hz, 2H); 3.8 (s, 3H); 5.5 (m, 1H); 6.1 (br s, 1H) ppm.

EXAMPLE 14

Methyl 2-(2-propyl)acrylate

This compound was prepared as above in approximately 10% overall yield from dimethyl malonate and isopropyl bromide. bp.=130-132°C at $P_{atm}$; NMR ($CDCl_3$) (60 MHz): 1.1 (d, J=7Hz, 6H); 2.8 (septet, 1H); 3.8 (s, 1H); 5.4 (t, 1H); 6.0 (br s, 1H) ppm.

EXAMPLE 15

Methyl (N-CBZ-1-amino-2-cyclohexyl)2-carbomethoxy-3-cyclohexylpropylphosphinate

A solution of 3.39 g (0.010 moles) of the product of Example 3 in 25 ml of absolute methanol was cooled to 0C under $N_2$. At this time, a solution of 5.50 ml of 2N NaOMe in methanol was added dropwise over a period of ca. 1 min. The reaction mixture was stirred for 10 min. and at this time, 1.91 g (0.0105 moles) of the product of Example 35

was added over a 1 min. period. The reaction mixture was stirred at 0°C for 30 min., then at room temperature for 23 hours. The volatiles were removed *in vacuo* and the residue added to 50 ml of ice-cold 1N HCl. The mixture was extracted with 3 X 50 ml of ethyl acetate. The combined ethyl acetate was dried over anh. $MgSO_4$, filtered, and the volatiles evaporated *in vacuo* to a colorless oil. The residue was chromatographed on silica gel using 18:1:1 methylene chloride:acetone:methanol as the eluant to afford 4.26 g (82%) of the desired product as a very viscous oil. NMR ($CDCl_3$) (300 MHz): 0.7-1.9 (m, 28H); 2.1 (m, 1H); 2.9 (br s, 1H); 3.6-3.8 (m, 6H); 4.1 (br s, 1H); 5.1 (s, 2H); 7.3 (s, 5H) ppm.

## EXAMPLE 16

### Methyl (1-amino-2-cyclohexyl)2-carbomethoxy-3-cyclohexylpropylphosphinate

A mixture of 4.00 g (7.7 mmol) of the product of Example 39 in 50 ml of absolute methanol was treated with 1.00 g of 10% Pd on C and hydrogenated in a Parr apparatus at 50 psig of $H_2$ for 20 hours. The catalyst was filtered off through celite and the pad washed well with methanol. The filtrate was evaporated *in vacuo* and the residue was chromatographed on silica gel using 18:1:1 methylene chloride:acetone:methanol as the eluant. This afforded 2.20 g (74%) of the desired product as a viscous oil. NMR ($CDCl_3$) (300 MHz): 0.7-1.9 (m, 28H); 2.1 (t, 1H); 2.2 (m, 1H); 2.9 (br s, 2H); 3.7 (m, 6H) ppm.

## EXAMPLE 17

Methyl 2-(2-methylpropyl)acrylate

This compound was prepared by the method of J. Harley-Mason *et al*. (*Tetrahedron* 1980, 36, 1063) in approximately 35% overall yield. NMR ($CDCl_3$) (300 MHz): 0.9 (d, 6H); 1.8 (septet, 1H); 2.2 (d, 2H); 3.7 (s, 3H); 5.5 (d, 1H); 6.1 (d, 1H) ppm.

## EXAMPLE 18

Methyl (N-CBZ-1-amino-2-cyclohexylethyl) carbomethoxymethylphosphinate

To a mixture of benzylcarbamate (8.66 g, 0.057 mol), pivalic acid (11.72 g, 0.11 mol) and 11 g of predried powdered molecular sieves in 120 ml of dry toluene was added carbomethoxymethyldichlorophosphine (10 g, 0.057 mol). The reaction mixture was cooled to 0°C and to it was added dropwise cyclohexylacetaldehyde (7.2 g, 0.057 mol). After stirring for 30 minutes at 0°C and 2 hours at room temperature, the reaction was filtered and the solvents removed by evaporation *in vacuo*. The residue was redissolved in 100 ml of dichloromethane cooled to 0°C and esterified with an ether solution of diazomethane. The solvents and excess diazomethane were subsequently removed by evaporation *in vacuo* and the crude product was purified by chromatography to give 11 g of the title compound.

Chromatography: silica, ethyl acetate

TLC (silica, ethyl acetate) $R_f$=0.48

NMR ($CDCl_3$, TMS) 0.9-2.0 (m, 13H), 2.95 (d 16Hz, 2H), 3.64 (s, 3H), 3.7 (d 12Hz, 3H), 3.9-4.5 (m.1H), 5.1 (s,2H), 5.4 and 5.8 (d 10Hz, 1H), 7.2 (s.5H)

mass spectrum: $M^+$

EXAMPLE 19

Methyl (N-CBZ-1-amino-3-methylbutyl) carbomethoxymethylphosphinate

This ester is prepared by the procedure described in Example 33, using 3-methylbutyraldehyde in place of cyclohexylacetaldehyde. It can also be prepared by the method of P. A. Bartlett *et al.*, *J. Amer. Chem. Soc.*, 106, 4282-83 (1984).

EXAMPLE 20

N-CBZ-1-aminoethylphosphinic acid

The title compound was prepared using the procedure described in Example 2 with paraldehyde replacing hexahydrophenylacetaldehyde.

EXAMPLE 21

Diethyl 2-(N-Benzyl)amino-3-phenylethylphosphonate

A solution of 45.80 g (0.427 moles) of benzylamine in 50 ml of methylene chloride was agitated during the addition of 51.35 g (0.427 moles) of freshly distilled phenylacetaldehyde. After the addition was half-complete, the reaction mixture became cloudy, and after completion of addition the mixture turned a yellow color. At this time, 40 g of anhydrous $Na_2SO_4$ was added and the reaction mixture was stirred at room temperature for 1 hour. The drying agent was filtered off and the filtrate evaporated *in vacuo* to a yellow liquid.

At this time, 56.02 g (0.406 moles) of diethylphosphite was added to the yellow liquid and the mixture was heated in an oil bath maintained at 170°C. The solution was maintained at this

temperature for 2.5 hours. The reaction mixture was cooled to room temperature and purified in two approximately equal portions by chromatography on 600 g of silica gel using 2:1 hexanes:ethyl acetate as the eluant. A more mobile impurity came off first, followed by the desired product as a thick yellow liquid. This procedure afforded 59.15 g (42%) of the diester. NMR ($CDCl_3$) (60 MHz): 1.0-1.6 (overlapping t, total=6H); 1.8 (br s, 2H); 2.0 (s, 2H); 2.5-3.5 (m, 2H); 3.7-4.5 (two overlapping q, 4H); 7.0 (m, 10H) ppm.

EXAMPLE 22

Diethyl 2-(N-Benzyl)amino-3-phenylethylphosphonate hydrochloride

A solution of 30.00 g (0.086 moles) of the product of Example 25 in 650 ml of anhydrous ether was treated with HCl gas for 45 minutes. The reaction was allowed to stand overnight and then filtered to remove a small amount of solid material. The remaining 29.15 g (0.084 moles) of starting material was dissolved in 430 ml of anhydrous ether and treated similarly. The combined filtrates from the two reactions was evaporated *in vacuo* and the viscous oil treated with several 200 ml portions of carbon tetrachloride followed by evaporation *in vacuo*. This procedure led to a sticky crystalline mass which was triturated with anhydrous ethyl ether and filtered and then vacuum dried to afford 41.55 g (64%) of the salt as a white crystalline solid. NMR ($CDCl_3$) (60 MHz): 1.0-1.6 (m, 6H); 2.1 (s, 2H); 3.2-4.5 (m, 9H); 7.2 (s, 10H) ppm.

EXAMPLE 23

Diethyl 2-Amino-3-phenylethylphosphonate hydrochloride

A solution of 2.00 g (0.0052 moles) of the product of Example 26 in 8 ml of absolute ethanol containing 0.200 g of 10% Pd on carbon was hydrogenated in a Parr type apparatus for 7 hours at 40 psig of hydrogen. The reaction mixture was filtered through a celite pad and the pad washed well with ethanol. The combined filtrates were evaporated *in vacuo* to give 1.50 g (99%) of the product as a very viscous, colorless oil. NMR ($d_2$-acetone) (60 MHz): 1.0-1.6 (overlapping t, 6H); 3.3-5.0 (m, 7H); 7.0-7.6 (m, 5H) ppm.

EXAMPLE 24

Diethyl 2-Amino-3-cyclohexylethylphosphonate hydrochloride

A solution of 1.50 g (0.0051 moles) of the product of Example 26 from a procedure similar to Example 27 in 20 ml of glacial acetic acid was treated with 1.50 g of $PtO_2$ and hydrogenated at 60°C and 50 psig of hydrogen in a Parr type of apparatus. After 2 hours, the reaction mixture was cooled to room temperature, filtered through a celite pad and the pad washed well with glacial acetic acid. The filtrate was evaporated completely *in vacuo* to give 1.34 g (86%) of essentially pure product. NMR ($CDCl_3$) (60 MHz): 0.8-2.2 (m, 19H); 3.6 (br s, 1H); 3.9-4.5 (m, 4H); 8.6 (v br s, 3H) ppm.

## EXAMPLE 25

**Diethyl N-[N-BOC-($N^{im}$-DNP)histidyl]-1-amino-2-cyclohexylethylphosphonate**

A solution of the product of Example 28 (0.400 g; 1.33 mmol), N-BOC-$N^{im}$-(2,4-DNP)histidine hydrate (0.586 g; 1.33 mmol), and 1-hydroxybenzotriazole hydrate (0.276 g; 1.6 mmol) in $CH_2Cl_2$ (15 ml) was cooled (ice-bath) and allowed to stir for 5 minutes. Dicyclohexylcarbodiimide (0.275 g; 1.6 mmol) was added and the mixture allowed to stir for 3 hours. The mixture was filtered, diluted with EtOAc (100 ml) and washed with 5% aqueous $NaHCO_3$ solution and brine, and dried ($Na_2SO_4$). Evaporation gave a crude product which was purified on silica gel (18:1:1 $CH_2Cl_2$:acetone:$CH_3OH$) giving the product (0.868 g) as a yellow solid. NMR ($CDCl_3$) (300 MHz): 0.7-1.8 (m containing 9H s at 1.4, total 28H); 3.0 (dd, 1H); 3.1 (dd, 1H); 4.0 (m, 4H); 4.5 (m, 2H); 6.1 (m, 1H), 7.0 (br d, 1H); 7.0 (d, 1H); 7.9 (dd, 1H); 8.6 (dt, 1H); 8.8 (m, 1H) ppm.

## EXAMPLE 26

**Diethyl N-[N-(N-CBZ-2-amino-3-(1-naphthyl)propionyl)-$N^{im}$-DNP-histidyl]-1-amino-2-cyclohexylethylphosphonate**

A mixture of the product of Example 9 (89 mg/ 0.256 mmol), disuccinimido oxalate (87 mg; 0.306 mmol), pyridine (24 mg; 0.306 mmol) and acetonitrile (10 ml) was allowed to stir for 24 hours. The phosphonate product of Example 30 (140 mg; 0.210 mmol) was dissolved in absolute EtOH (10 ml) and the solution saturated with anhydrous HCl. The residue after evaporation was dissolved in

$CH_2Cl_2$ (3 ml) and added to the above mixture. The combined mixture was allowed to stir for 24 hours, then diluted with EtOAc (50 ml), washed with 5% aqueous $NaHCO_3$, brine and dried ($Na_2SO_4$). Evaporation gave a crude product which was purified by chromatography on silica gel (18:1:1 $CH_2Cl_2$:acetone:$CH_3OH$) giving the product (120 mg; 0.133 mmol; 63%) as a white solid. NMR ($CD_3OD$): 0.7-1.8 (19H, m); 3.0-3.2 (2H, broad s); 3.2-3.7 (2H, m); 4.0-4.2 (4H, m); 4.3-4.6 (2H, m); 4.7-4.9 (1H, m); 4.95 (2H, s); 5.4-5.6 (1H, broad d, J=7); 7.0-8.8 (17H, m).

## EXAMPLE 27

Ethyl N-[N(N'-CBZ-2-amino-3-(1-naphthyl)propionyl)-$N^{im}$-DNP-histidyl]-1-amino-2-cyclohexylethylphosphonate

A mixture of the product of Example 31 (49.3 mg; 0.548 mmol) and aqueous NaOH (1.9 ml; 0.05 N) was stirred for 8 hours. Glacial acetic acid (7 µl) was added and the mixture evaporated to dryness. Chromatography on silica gel (18:5:1:1 $CHCl_3$:$CH_3OH$:$H_2O$:HOAc) gave product (12 mg; 0.017 mmol. 31%) as a light-yellow solid. NMR ($CD_3OD$): 0.7-2.1 (16H, m); 3.0-3.8 (4H, m); 4.2-4.4 (2H, m); 4.4-4.8 (3H, m); 5.9 (2H, 2 x s); 6.9-8.4 (14H, m).

## EXAMPLE 28

N-Methyl 1-amino-2-cyclohexylphosphinic acid

This acid is prepared using the procedure described in Example 2 with methylamine hydrochloride replacing aminophenylmethane hydrochloride.

EXAMPLE 29

Methyl N-CBZ-N-methyl 1-amino-2-cyclohexylethyl phosphinate

This ester is prepared from N-methyl-1-amino-2-cyclohexylphosphinic acid using the procedure described in Example 3.

EXAMPLE 30

Diethyl N-methyl-1-amino-2-phenylethylphosphonate

This ester is prepared using the procedure described in Example 21 with equimolar amounts of methyl amine hydrochloride and triethylamine substituted for benzylamine.

EXAMPLE 31

Diethyl N-CBZ-N-methyl-1-amino-2-cyclohexylethyl-phosphonate

This ester is prepared by treatment of the product of Example 30 with benzyl chloroformate and $Et_3N$ in $CH_2Cl_2$ and is purified by silica gel chromatography.

EXAMPLE 32

Diethyl N-CBZ-1-amino-2-cyclohexylethylphosphonate

This ester is prepared by treatment of the product of example 24 with benzyl chloroformate and $Et_3N$ in $CH_2Cl_2$ and is purified by silica gel chromatography.

EXAMPLE 33

N-Benzylhexahydrotriazine

To a solution of benzylamine (27 ml) in absolute ethanol (25 ml) was added rapidly an aqueous solution of formaldehyde (37%) (18.5 ml). The reaction began refluxing at this stage. The mixture was stirred at room temperature for 2 hours and then diluted with light petroleum ether (250 ml), washed with water (3 x 100 ml) and brine. The organic phase was evaporated to give a semisolid mass. The crude product was crystallized from petroleum ether to give white crystals, mp 49-51°C (24 g).

EXAMPLE 34

(N-CBZ-1-aminoethyl) N-benzylaminomethylphosphinic acid

A mixture of N-CBZ-1-aminoethylphosphonous acid (1 g) and the product of Example 33 (0.48 g) in dry toluene (10 ml) was heated at 120°C for 5 hours, cooled to room temperature, and diluted with EtOAc (30 ml). The white precipitate was collected by filtration, washed with EtOAc (5 ml) and dried, mp 232-233°C, yield 0.6 g. The product was a single spot on TLC (4:1:1 2-butanone:acetone:$H_2O$) and gave a parent ion in the FAB mass spectrum ($M^+$+1 363).

EXAMPLE 35

(N-CBZ-1-amino-2-cyclohexylethyl)(N-benzyl)aminomethylphosphinic acid

A mixture of N-CBZ-1-amino-2-cyclohexyl ethyl phosphinic acid prepared as described in Example 3 (0.325 g) and N-benzyl-s-triazine (0.12 g)

was suspended in toluene (4 ml) and refluxed for 4 hours. After standing at 25°C for an additional 15 hours, the reaction mixture was concentrated in vacuo giving an oil. The oil was dissolved in minimum volume of acetone and filtered. The filtrate was evaporated in vacuo giving a semisolid mass. The product had a satisfactory NMR and mass spectral data. Yield 0.35 g.

EXAMPLE 36

(N-CBZ-1-amino-2-cyclohexylethyl)(N-benzyl)-N-BOC-aminomethylphosphinic acid

A mixture of the product of Example 35 (0.3 g), $BOC_2O$ (0.18 g) and $Et_3N$ (0.094 ml) in $CH_3OH$ (3 ml) was stirred at 25°C for 15 hours. Removal of the solvent gave the crude product as viscous oil which was purified by MPLC using ethylacetate containing 3% MeOH. Yield 0.26 g (foam).

EXAMPLE 37

N,N'N"-Tris(diphenylmethyl)hexahydro-s-triazine

To a solution of aminodiphenylmethane (10 ml, 0.0513 mol) in absolute EtOH (20 ml) was added rapidly with stirring, aqueous formaldehyde (37%) (3.75 ml, 0.0462 mol). The temperature of the resulting clear solution rose to 60°C, and a white precipitate separated out within 1 minute. The mixture was diluted with absolute EtOH (10 ml) and refluxed for 2 hours. The reaction mixture was cooled with an ice bath, and the solid was filtered and washed with cold EtOH (5 ml). Recrystallization from hot methanol gave product (12.5 g), mp 236-237°C.

EXAMPLE 38

(N-CBZ-1-amino-2-cyclohexylethyl) N-(diphenylmethyl)-aminomethylphosphinic acid

A mixture of N-CBZ-1-amino-2-cyclohexylethylphosphonous acid (Example 3) (0.325 g, 1.00 mmol) and the triazine product of Example 37 (0.195, 9.33 mmol) was suspended in dry toluene (5 ml) and refluxed for 4 hours, cooled to room temperature, and treated with saturated $NaHCO_3$ solution. The basic aqueous layer was acidified with acetic acid to pH = 5.0 and extracted with EtOAc (30 ml). The organic phase was washed with brine, dried ($MgSO_4$) and evaporated, giving the product as a foam. The product was found to be a single spot by TLC (silica gel: 2-butanone:acetone:$H_2O$, 9:1:1).

Claims to the invention follow.

WHAT IS CLAIMED IS:

1. A compound of the formula:

A-B-D-E-G

wherein:

A is hydrogen, or R-, RCO- or $RSO_2$-, where R is alkyl, cycloalkyl, aryl, heterocyclic, heterocyclic alkyl, heterocyclic aryl, aryloxy alkyl, heterocyclic aryloxy alkyl, aryl alkyl, heterocyclic aryl alkyl, heterocyclic oxyalkyl, and R- may be substituted by amino, hydroxy, alkyl, halo, and alkoxy groups.

B and D can independently be absent or can be:

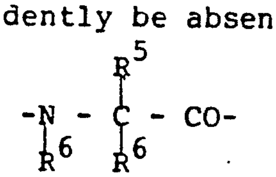

II

E is

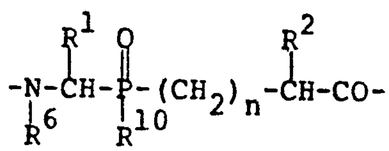

III

or

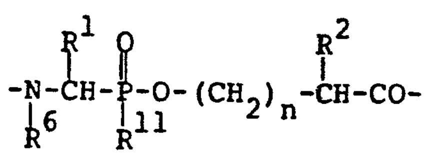

IV

or, in the case where G is absent, E may be:

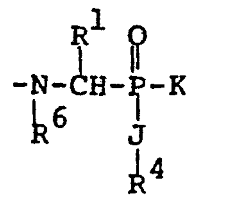

V

E may also be:

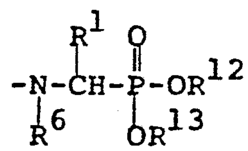

VI

G is $R^3$ or is:

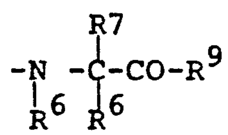

J is -O- or, in the case where $R^4$ does not equal hydrogen, may be absent.

K is hydrogen, alkyl, alkenyl, alkynyl, cycloalkyl, cycloalkenyl, aryl alkyl, cycloalkyl alkyl, heterocyclic, heterocyclic alkyl, each of which may be substituted by up to three members selected from amino, alkyl amino, dialkyl amino, trialkyl ammonium, hydroxy, alkoxy, aryloxy, arylalkoxy, alkanoylamino, aroylamino, or halo.

K may also be

$$-(CH_2)_{n'}-CO-R^3$$

where n' is 3 to 5 and $R^3$ is as defined below.

K may also be of the formula:

$$-(CH_2)_p-NH-L$$

where p is 1 to 2 and

L is $-\underset{R^5}{\underset{|}{C}}H-CO-R^3$,

$-CO-\underset{R^5}{\underset{|}{C}}H-NH_2$, or A as defined above.

$R^1$ is alkyl, alkenyl, alkynyl, cycloalkyl, cycloalkenyl, cycloalkyl alkyl, aryl alkyl, heterocyclic, heterocyclic alkyl, each of which may be substituted by up to three groups selected from alkyl, halo, amino and alkoxy groups.

n is 0, 1.

$R^2$ is hydrogen, alkyl, alkenyl, alkynyl, cycloalkyl, cycloalkenyl, cycloalkyl alkyl, aryl, aryl alkyl, heterocyclic, heterocyclic alkyl, each of which may be substituted by up to three groups selected from alkyl, hydroxy, halo, amino, alkylamino, dialkylamino, and alkoxy groups.

$R^3$ is OH, $NH_2$, $NHR^3_a$, $NR^3_aR^3_b$, $OR^3_c$ where $R^3_a$, $R^3_b$, and $R^3_c$ are separately alkyl, alkenyl, alkynyl, cycloalkyl, cycloalkenyl, cycloalkyl alkyl, aryl, aryl alkyl, heterocyclic, heterocyclic

alkyl, each of which may be substituted by up to three groups selected from amino, alkyl amino, dialkyl amino, trialkyl ammonium, hydroxy, alkoxy, aryloxy, aryl alkoxyl, or halo.

$R^3_c$ may also be $R^3_d$-CO-V-$CR^3_eR^3_f$ wherein $R^3_d$ is alkyl or aryl; $R^3_e$ and $R^3_f$ are hydrogen or alkyl; V is -O- or -NH-.

$R^4$ is hydrogen, alkyl, alkenyl, alkynyl, aryl, arylalkyl, each of which may be substituted by amino, alkyl amino, dialkyl amino, trialkyl ammonium, hydroxy, alkoxy, halo or alkyl groups. $R^4$ may also be $R^4_a$-CO-V-$CR^4_bR^4_c$ wherein $R^4_a$ is alkyl or aryl; $R^4_b$ and $R^4_c$ are hydrogen or alkyl; V is -O- or -NH-.

$R^5$ is hydrogen, alkyl, alkenyl, alkynyl, cycloalkyl, cycloalkenyl, aryl, cycloalkyl alkyl, aryl alkyl, heterocyclic, heterocyclic alkyl, aryloxy alkyl, heterocyclic oxy alkyl, heterocyclic oxy oxy, each of which may be substituted by amino, alkyl amino, dialkyl amino, trialkyl ammonium, hydroxy, alkoxy, carboxy, alkoxycarbonyl, alkylthio, arylthio, thiol, guanidino, carboxamido and alkanoylamino groups.

$R^6$ is hydrogen, methyl.

$R^7$ can be $R^5$ and taken together with $NR^8$ may be a cyclic amino acid of formulas:

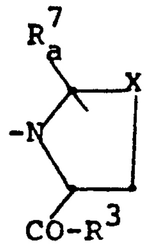 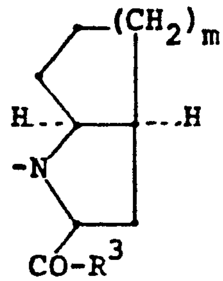 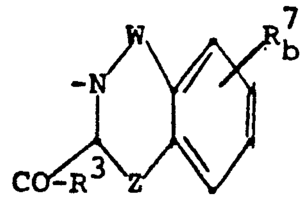

where $R_a^7$ is hydrogen, phenyl, hydroxyphenyl; X is -S- or $-CH_2-$ or $-CH-R_b^7$; m is 1 or 2; and $R_b^7$ is cyclohexyl, phenylthio; W and Z are bonds or $-CH_2-$, and $R^3$ is as defined above.

$R^8$ is hydrogen, methyl and cycloalkyl including cyclopentyl and indanyl. When $R^8$ is cycloalkyl, $R^6$ and $R^7$ are hydrogen.

$R^9$ is hydroxy, $OR_a^3$, $-NH_2$, $-NHR_a^3$, $NR_a^3R_b^3$, where $R_a^3$ and $R_b^3$ are as defined above. When A and B are both absent, $R^9$ can be 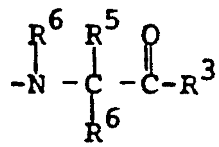 where $R^3$, $R^5$ and $R^6$ are as defined above.

$R^{10}$ is alkyl, aryl alkyl, heterocyclic, heterocyclic alkyl, where each of these may be substituted by up to three groups selected from amino, alkyl amino, dialkyl amino, trialkyl ammonium, hydroxy, alkoxy, thiol, carboxamido, and alkanoylamino or aroylamino groups.

$R^{11}$ is hydrogen or $-OR^4$, where $R^4$ is as defined above.

$R^{12}$ and $R^{13}$ may independently be hydrogen, alkyl, alkenyl, alkynyl, aryl, aryl alkyl, each of which may be substituted by up to three groups selected from hydroxy, alkyl, amino, alkyl amino, dialkyl amino, trialkyl ammonium, halo, alkanoylamino, aroylamino, alkoxy, and aryloxy groups;

and pharmaceutically acceptable salts thereof.

2. A compound of Claim 1 wherein the B and D units have the L-isomer form.

3. A compound of Claim 1 wherein the E unit has the L-isomer configuration.

4. A compound of Claim 1 selected from:

1. [N-(N-(N-Carbobenzoxy-2-amino-3-(1-naphthyl)-propionyl)histidyl)-1-amino-2-cyclohexylethyl] 2-carboxy-4-methylpentyl methyl phosphine oxide
2. [N-(N-(N-Carbobenzoxy-2-amino-3-(1-naphthyl)-propionyl)histidyl)-1-amino-2-cyclohexylethyl] 2-carboxy-3-methylbutylphosphine oxide
3. 2-Carboxy-3-methylbutyl [N-(N-(N-t-butoxycarbonyl-2-amino-3-(1-naphthyl)propionyl)histidyl)-1-amino-2-cyclohexylethyl]phosphinate
4. 2-Carbomethoxy-4-methylpentyl [N-(N-(N-carbobenzoxy-2-amino-3-(1-naphthyl)propionyl)histidyl)-1-amino-2-cyclohexylethyl] methylphosphinate
5. [N-(N-(N-Carbobenzoxy-2-amino-3-(1-naphthyl)-propionyl)histidyl)-1-amino-2-cyclohexylethyl]-phosphonic acid
6. Ethyl [N-(N-(N-t-butoxycarbonyl-2-amino-3-(1-naphthyl)propionyl)histidyl)-1-amino-2-cyclohexylethyl]phosphonate

7. 2-Carboxy-4-methylpentyl methyl [N-(N-carbobenzoxy-phenylalanyl-histidyl)-1-amino-2-cyclohexylethyl] phosphonate
8. [N-(N-Carbobenzoxy-phenylalanyl-histidyl)-1-amino-2-cyclohexylethyl] 2-(N-benzyl)carboxamido-4-methylpentyl benzyl phosphine oxide
9. 2-Carboxamido-4-methylpentyl [N-(N-carbobenzoxy-phenylalanyl-phenylalanyl)-1-amino-2-cyclohexylethyl]phosphinate
10. [N-(N-(N-Carbobenzoxy-2-amino-3-(1-naphthyl)-propionyl)histidyl)-1-amino-2-cyclohexylethyl] 3-methylbutylphosphinic acid
11. Methyl [N-(N-(N-carbobenzoxy-2-amino-3-(1-naphthyl)propionyl)histidyl)-1-amino-2-cyclohexylethyl] 4-methylpentylphosphinate
12. [N-(N-(N-Carbobenzoxy-2-amino-3-(1-naphthyl)-propionyl)histidyl)-1-amino-2-cyclohexylethyl] aminomethylphosphinic acid
13. [N-(N-(N-Carbobenzoxy-2-amino-3-(1-naphthyl)-propionyl)histidyl)-1-amino-2-cyclohexylethyl] (N-benzoyl)aminomethylphosphinic acid
14. [N-(N-Carbobenzoxy-histidyl)-1-amino-2-cyclohexylethyl] 4-methylpentylphosphinic acid
15. [N-(Phenylalanyl)-1-amino-2-cyclohexylethyl]-3-methylbutylphosphinic acid
16. [N-(Lysyl)-1-amino-2-cyclohexylethyl]-3-methylbutylphosphinic acid
17. [N-(N-1-Naphthyloxyacetyl-lysyl)-1-amino-2-cyclohexylethyl] (n-benzyl)aminomethylphosphinic acid
18. Methyl [N-(N-(N-carbobenzoxy-2-amino-3-(1-naphthyl)propionyl)histidyl)-1-amino-2-cyclohexylethyl] 4-phenylpentylphosphinate

5. A pharmaceutical composition for treating hypertension and congestive heart failure containing a compound of Claim 1.

6. A pharmaceutical composition of Claim 5 additionally containing another antihypertensive agent.

CLAIMS FOR AUSTRIA:

1. A process for preparing a compound of the formula:

A-B-D-E-G

wherein:

A is hydrogen, or R-, RCO- or $RSO_2$-, where R is alkyl, cycloalkyl, aryl, heterocyclic, heterocyclic alkyl, heterocyclic aryl, aryloxy alkyl, heterocyclic aryloxy alkyl, aryl alkyl, heterocyclic aryl alkyl, heterocyclic oxyalkyl, and R- may be substituted by amino, hydroxy, alkyl, halo, and alkoxy groups.

B and D can independently be absent or can be:

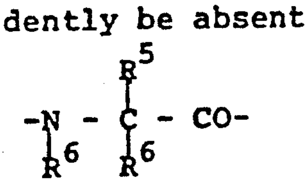

II

E is

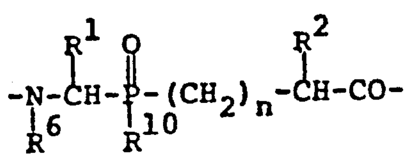

III

or

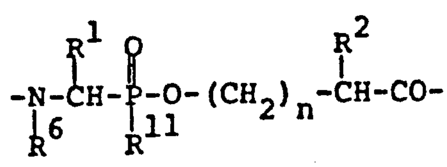

IV

or, in the case where G is absent, E may be:

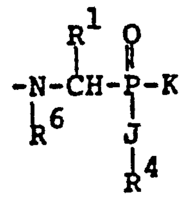

V

E may also be:

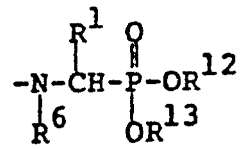

VI

G is $R^3$ or is:

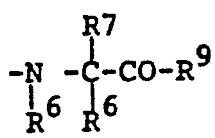

J is -O- or, in the case where $R^4$ does not equal hydrogen, may be absent.

K is hydrogen, alkyl, alkenyl, alkynyl, cycloalkyl, cycloalkenyl, aryl alkyl, cycloalkyl alkyl, heterocyclic, heterocyclic alkyl, each of which may be substituted by up to three members selected from amino, alkyl amino, dialkyl amino, trialkyl ammonium, hydroxy, alkoxy, aryloxy, arylalkoxy, alkanoylamino, aroylamino, or halo.

K may also be

$$-(CH_2)_{n'}-CO-R^3$$

where n' is 3 to 5 and $R^3$ is as defined below.

K may also be of the formula:

$$-(CH_2)_p-NH-L$$

where p is 1 to 2 and

L is $-\underset{R^5}{\underset{|}{CH}}-CO-R^3$,

$-CO-\underset{R^5}{\underset{|}{CH}}-NH_2$, or A as defined above.

$R^1$ is alkyl, alkenyl, alkynyl, cycloalkyl, cycloalkenyl, cycloalkyl alkyl, aryl alkyl, heterocyclic, heterocyclic alkyl, each of which may be substituted by up to three groups selected from alkyl, halo, amino and alkoxy groups.

n is 0, 1.

$R^2$ is hydrogen, alkyl, alkenyl, alkynyl, cycloalkyl, cycloalkenyl, cycloalkyl alkyl, aryl, aryl alkyl, heterocyclic, heterocyclic alkyl, each of which may be substituted by up to three groups selected from alkyl, hydroxy, halo, amino, alkylamino, dialkylamino, and alkoxy groups.

$R^3$ is OH, $NH_2$, $NHR^3_a$, $NR^3_aR^3_b$, $OR^3_c$ where $R^3_a$, $R^3_b$, and $R^3_c$ are separately alkyl, alkenyl, alkynyl, cycloalkyl, cycloalkenyl, cycloalkyl alkyl, aryl, aryl alkyl, heterocyclic, heterocyclic

alkyl, each of which may be substituted by up to three groups selected from amino, alkyl amino, dialkyl amino, trialkyl ammonium, hydroxy, alkoxy, aryloxy, aryl alkoxyl, or halo.

$R_c^3$ may also be $R_d^3$-CO-V-$CR_e^3R_f^3$ wherein $R_d^3$ is alkyl or aryl; $R_e^3$ and $R_f^3$ are hydrogen or alkyl; V is -O- or -NH-.

$R^4$ is hydrogen, alkyl, alkenyl, alkynyl, aryl, arylalkyl, each of which may be substituted by amino, alkyl amino, dialkyl amino, trialkyl ammonium, hydroxy, alkoxy, halo or alkyl groups. $R^4$ may also be $R_a^4$-CO-V-$CR_b^4R_c^4$ wherein $R_a^4$ is alkyl or aryl; $R_b^4$ and $R_c^4$ are hydrogen or alkyl; V is -O- or -NH-.

$R^5$ is hydrogen, alkyl, alkenyl, alkynyl, cycloalkyl, cycloalkenyl, aryl, cycloalkyl alkyl, aryl alkyl, heterocyclic, heterocyclic alkyl, aryloxy alkyl, heterocyclic oxy alkyl, heterocyclic oxy oxy, each of which may be substituted by amino, alkyl amino, dialkyl amino, trialkyl ammonium, hydroxy, alkoxy, carboxy, alkoxycarbonyl, alkylthio, arylthio, thiol, guanidino, carboxamido and alkanoylamino groups.

$R^6$ is hydrogen, methyl.

$R^7$ can be $R^5$ and taken together with $NR^8$ may be a cyclic amino acid of formulas:

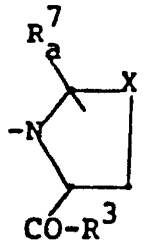
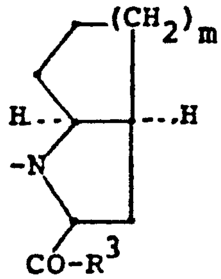
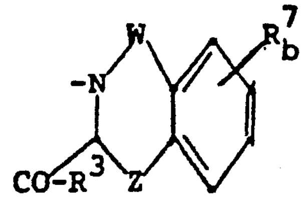

where $R_a^7$ is hydrogen, phenyl, hydroxyphenyl; X is -S- or $-CH_2-$ or $-CH-R_b^7$; m is 1 or 2; and $R_b^7$ is cyclohexyl, phenylthio; W and Z are bonds or $-CH_2-$, and $R^3$ is as defined above.

$R^8$ is hydrogen, methyl and cycloalkyl including cyclopentyl and indanyl. When $R^8$ is cycloalkyl, $R^6$ and $R^7$ are hydrogen.

$R^9$ is hydroxy, $OR_a^3$, $-NH_2$, $-NHR_a^3$, $NR_a^3R_b^3$, where $R_a^3$ and $R_b^3$ are as defined above. When A and B are both absent, $R^9$ can be 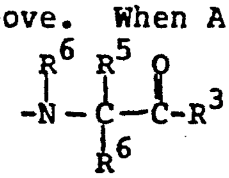 where $R^3$, $R^5$ and $R^6$ are as defined above.

$R^{10}$ is alkyl, aryl alkyl, heterocyclic, heterocyclic alkyl, where each of these may be substituted by up to three groups selected from amino, alkyl amino, dialkyl amino, trialkyl ammonium, hydroxy, alkoxy, thiol, carboxamido, and alkanoylamino or aroylamino groups.

$R^{11}$ is hydrogen or $-OR^4$, where $R^4$ is as defined above.

$R^{12}$ and $R^{13}$ may independently be hydrogen, alkyl, alkenyl, alkynyl, aryl, aryl alkyl, each of which may be substituted by up to three groups selected from hydroxy, alkyl, amino, alkyl amino, dialkyl amino, trialkyl ammonium, halo, alkanoylamino, aroylamino, alkoxy, and aryloxy groups;

and pharmaceutically acceptable salts thereof comprising

a) coupling of an amino-protected form of D to E-G, followed by amino protecting group removal from D-E-G and coupling of A-B to the deprotected amino group of D-E-G or

b) coupling of A-B-D to E-G by known coupling procedures, functional groups present which do not participate in the coupling reaction being protected in a manner known per se prior to the coupling reaction and deprotected in a manner known per se after the coupling reaction, and, if desired, the compound obtained being converted in a manner known per se to a pharmaceutically acceptable salt.

2. The process of Claim 1 for preparing a compound wherein the B and D units have the L-isomer form.

3. The process of Claim 1 for preparing a compound wherein the E unit has the L-isomer configuration.

4. The process of Claim 1 for preparing a compound selected from:

1. [N-(N-(N-Carbobenzoxy-2-amino-3-(1-naphthyl)-propionyl)histidyl)-1-amino-2-cyclohexylethyl] 2-carboxy-4-methylpentyl methyl phosphine oxide
2. [N-(N-(N-Carbobenzoxy-2-amino-3-(1-naphthyl)-propionyl)histidyl)-1-amino-2-cyclohexylethyl] 2-carboxy-3-methylbutylphosphine oxide

3. 2-Carboxy-3-methylbutyl [N-(N-(N-t-butoxycarbonyl-2-amino-3-(1-naphthyl)propionyl)histidyl)-1-amino-2-cyclohexylethyl]phosphinate
4. 2-Carbomethoxy-4-methylpentyl [N-(N-(N-carbobenzoxy-2-amino-3-(1-naphthyl)propionyl)histidyl)-1-amino-2-cyclohexylethyl] methylphosphinate
5. [N-(N-(N-Carbobenzoxy-2-amino-3-(1-naphthyl)-propionyl)histidyl)-1-amino-2-cyclohexylethyl]-phosphonic acid
6. Ethyl [N-(N-(N-t-butoxycarbonyl-2-amino-3-(1-naphthyl)propionyl)histidyl)-1-amino-2-cyclohexylethyl]phosphonate
7. 2-Carboxy-4-methylpentyl methyl [N-(N-carbobenzoxy-phenylalanyl-histidyl)-1-amino-2-cyclohexylethyl] phosphonate
8. [N-(N-Carbobenzoxy-phenylalanyl-histidyl)-1-amino-2-cyclohexylethyl] 2-(N-benzyl)carboxamido-4-methylpentyl benzyl phosphine oxide
9. 2-Carboxamido-4-methylpentyl [N-(N-carbobenzoxy-phenylalanyl-phenylalanyl)-1-amino-2-cyclohexylethyl]phosphinate
10. [N-(N-(N-Carbobenzoxy-2-amino-3-(1-naphthyl)-propionyl)histidyl)-1-amino-2-cyclohexylethyl] 3-methylbutylphosphinic acid
11. Methyl [N-(N-(N-carbobenzoxy-2-amino-3-(1-naphthyl)propionyl)histidyl)-1-amino-2-cyclohexylethyl] 4-methylpentylphosphinate
12. [N-(N-(N-Carbobenzoxy-2-amino-3-(1-naphthyl)-propionyl)histidyl)-1-amino-2-cyclohexylethyl] aminomethylphosphinic acid
13. [N-(N-(N-Carbobenzoxy-2-amino-3-(1-naphthyl)-propionyl)histidyl)-1-amino-2-cyclohexylethyl] (N-benzoyl)aminomethylphosphinic acid
14. [N-(N-Carbobenzoxy-histidyl)-1-amino-2-cyclohexylethyl] 4-methylpentylphosphinic acid

15. [N-(Phenylalanyl)-1-amino-2-cyclohexylethyl]-3-methylbutylphosphinic acid
16. [N-(Lysyl)-1-amino-2-cyclohexylethyl]-3-methylbutylphosphinic acid
17. [N-(N-1-Naphthyloxyacetyl-lysyl)-1-amino-2-cyclohexylethyl] (n-benzyl)aminomethylphosphinic acid
18. Methyl [N-(N-(N-carbobenzoxy-2-amino-3-(1-naphthyl)propionyl)histidyl)-1-amino-2-cyclohexylethyl] 4-phenylpentylphosphinate .